# EUROPEAN PATENT SPECIFICATION

(11) **EP 1 147 134 B1**
(45) Date of publication and mention of the grant of the patent: **11.10.2006**
(21) Application number: 00901048.9
(22) Date of filing: 19.01.2000
(51) Int. Cl.: C07K 14/705, C12N 15/12, A01K 67/027, C07K 16/28

(54) **NOVEL POTASSIUM CHANNELS AND GENES ENCODING THESE POTASSIUM CHANNELS**
KALIUMKANÄLE UND FÜR DIESE KALIUMKANÄLE KODIERENDE GENE
NOUVEAUX CANAUX POTASSIUM ET GENES CODANT POUR CES CANAUX POTASSIUM

(30) Priority: 26.01.1999 DK 990076; 19.05.1999 DK 990693
(43) Date of publication of application: 24.10.2001
(73) Proprietor: NEUROSEARCH A/S, 2750 Ballerup (DK)
(72) Inventor: JENTSCH, Thomas J., Falkenried 94 20251 Hamburg (DE)
(86) International application number: PCT/DK2000/000024
(87) International publication number: WO 2000/044786

(56) References cited:
- WO-A-97/23598
- WO-A-99/21875
- DATABASE MEDLINE [Online] ; KUBISCH C ET AL: "KCNQ4, a novel potassium channel expressed in sensory outer hair cells, is mutated in dominant deafness" retrieved from MEDLINE, accession no. 99148276 XP002900983 & CELL, vol. 96, no. 3, 5 February 1999 (1999-02-05), pages 437-446,
- NANDA A SINGH ET AL: "A novel potassium channel gene, KCNQ2, is mutated in an inherited epilepsy of newborns" NATURE GENETICS, vol. 18, January 1998 (1998-01), pages 25-29, XP002900984
- HONG-SHENG WANG ET AL: "KCNQ2 and KCNQ3 potassium channel subunits: Molecular correlates of the M-channel" SCIENCE, vol. 282, 4 December 1998 (1998-12-04), pages 1890-1893, XP002900985
- BIERVERT C ET AL: "A potassium channel mutation in neonatal human epilepsy" SCIENCE, vol. 279, 16 January 1998 (1998-01-16), pages 403-406, XP002900986
- DATABASE MEDLINE [Online] ; TAM SW ET AL: "Limopirdine. A depolarization-activated releaser of transmitters for treatment of dementia" retrieved from MEDLINE, accession no. UI: 95343820 XP002900987 & ADV EXP MED BIOL, no. 363, 1995, pages 47-56,

## Description

### TECHNICAL FIELD

This invention relates to novel potassium channels and genes encoding these channels. More specifically the invention provides isolated polynucleotides encoding the KCNQ4 potassium channel subunit, cells transformed with these polynucleotides, transgenic animals comprising genetic mutations, and the use of the transformed cells and the transgenic animals for the *in vitro* and *in vivo* screening of chemical compounds affecting KCNQ4 subunit containing potassium channels.

### BACKGROUND ART

Potassium channels participate in the regulation of electrical signalling in excitable cells, and regulate the ionic composition of biological fluids. Mutations in the three known genes of the *KCNQ* branch of the K⁺-channel gene family underlie inherited cardiac arrhythmia's, in some cases associated with deafness, and neonatal epilepsy.

Hearing loss is the most frequent sensory defect in humans. Hearing loss can be due to environmental and genetic factors, and the progressive hearing loss of the elderly (presbyacusis) most often seems to be due to a combination of both.

Inherited deafness can be classified as non-syndromic (isolated hearing loss) or syndromic (associated with other anomalies). Several hundred syndromes, consisting of hearing loss associated with defects in a variety of other organ systems, have been described. Non-syndromic deafness is classified according to its mode of inheritance as DFN, DFNA, and DFNB (X-linked, autosomal dominant and autosomal recessive, respectively). In general, autosomal recessive deafness has an early onset and is very severe. Autosomal dominant deafness, by contrast, more often develops slowly over several decades and may become apparent only in adulthood. It is hoped that genes identified in families with dominant deafness may also - with different types of mutations - underlie some forms of presbyacusis.

A bewildering number of loci for non-syndromic deafness were identified in the last four years. There are at least 19 loci for autosomal dominant deafness (DFNA1 to DFNA19), and 22 loci for DFNB. Sometimes, depending on the particular mutation, the same gene can be involved in dominant or recessive deafness. This large number of loci reflects the complexity of the inner ear. Identification of these genes and characterisation of their products will significantly advance our understanding of the molecular basis of the physiology of this sensory organ.

Several genes involved in syndromic and non-syndromic deafness have already been identified and are reviewed by *Petit* [*Petit C*: Genes responsible for human hereditary deafness: symphony of a thousand; Nature Genet. 1996 **14** 385-391] and *Kalatzis & Petit [Kalatzis V & Petit C:* The fundamental and medical impacts of recent progress in research on hereditary hearing loss; Hum. Mol. Genet. 1998 **7** 1589-1597]. Among others, their gene products include transcription factors, unconventional myosin isoforms, α-tectorin (an extracellular matrix protein), diaphanous, a protein interacting with the cytoskeleton, connexin 26 (a gap junction protein), and two genes encoding potassium channel subunits, *KCNQ1* and *KCNE1.*

Ion channels play important roles in signal transduction and in the regulation of the ionic composition of intra- and extracellular fluids. Mutations in ion channels were since long suspected as possibly underlying some forms of hearing loss. In the cochlea (the auditory sensory organ), the transduction current through the sensory cells is carried by potassium ions and depends on the high concentration of that ion in the endolymph. So far only two genes encoding potassium channel subunits, *KCNQ1* and *KCNE1,* were found to be mutated in syndromic hereditary deafness. The gene products of both genes, the KCNQ1 (or KvLQT1) and the minK (or IsK) protein, respectively, form heteromeric potassium channels.

KCNQ1 is a typical member of the voltage-gated potassium channel superfamily with 6 transmembrane domains and a pore region situated between the fifth and the sixth transmembrane domain. The minK protein has a single transmembrane span and cannot form potassium channels on its own. However, as a β-subunit it enhances and modifies currents mediated by KCNQ1. These heteromeric channels participate in the repolarization of the heart action potential. Certain mutations in either *KCNQ1* or *KCNE1* cause a form of the autosomal dominant long QT syndrome (LQTS), a disease characterised by repolarization anomalies of cardiac action potentials resulting in arrhythmias and sudden death. Interestingly, other mutations in either gene lead to the recessive Jervell and Lange-Nielsen (JLN) syndrome that combines LQTS with congenital deafness. In order to cause deafness, KCNQ1/minK currents must be reduced below levels that are already sufficiently low to cause cardiac arrhythmia.

WO 97/23598 discloses a long QT syndrome gene encoding KVLQT1 (i.e. KCNQ1) and its association with minK.

*Nanda A Singh et al.;* Nature Genetics 1998 **18** 2 5-29, describe the potassium channel KCNQ2 gene and describes mutations herein associated with inherited epilepsy.

*Hong-Sheng Wang et al.;* Science 1998 **282** 1890-1893, describe KCNQ2 and KCNQ3 potassium channel subunits.

### SUMMARY OF THE INVENTION

We have now cloned and characterised KCNQ4, a novel member of the KCNQ family of potassium channel proteins. *KCNQ4* has been mapped to the DFNA2 locus for autosomal dominant hearing loss, and a dominant negative *KCNQ4* mutation that causes deafness in a DFNA2 pedigree was identified.

*KCNQ4* is the first potassium channel gene underlying non-syndromic deafness. KCNQ4 forms heteromeric channels with other KCNQ channel subunits, in particular KCNQ3.

The present invention has important implications for the characterisation and exploitation of this interesting branch of the potassium channel super family, as well as for the understanding of the cochlear physiology, and for human deafness and progressive hearing loss.

Accordingly, in its first aspect, the invention provides an isolated polynucleotide having a nucleic acid sequence which is capable of hybridising under high stringency conditions with the polynucleotide sequence presented as SEQ ID NO: 1, or its complementary strand, wherein said hybridization occurs in a solution of 5 x SSC, 5 x Denhardt's solution, 0.5 % SDS and 100 µg/ml of denatured sonicated salmon sperm DNA for 12 hours at approximately 45°C, followed by washing twice for 30 minutes in 2 x SSC, 0.5 % SDS at a temperature of at least 65°C, encoding a potassium channel KCNQ4 subunit.

In another aspect the invention provides a recombinantly produced polypeptide encoded by the polynucleotide of the invention.

In a third aspect the invention provides a cell genetically manipulated by the incorporation of a heterologous polynucleotide of the invention.

In a fourth aspect the invention provides a method of screening a chemical compound for its capability of binding to, or activity on a potassium channel comprising at least one KCNQ4 channel subunit.

In a fifth aspect the invention relates to the use of a polynucleotide sequence of the invention for the screening of genetic materials from humans suffering from loss of hearing (e.g. dominant, recessive, or otherwise), tinnitus, and other neurological diseases for mutations in the *KCNQ4* gene.

In a sixth aspect the invention relates to the chemical compound identified by the method of the invention, in particular to the use of such compounds for diagnosis, treatment or alleviation of a disease related to tinnitus; loss of hearing, in particular progressive hearing loss, neonatal deafness, and presbyacusis (deafness of the elderly); and diseases or adverse conditions of the CNS, including affective disorders, Alzheimer's disease, anxiety, ataxia, CNS damage caused by trauma, stroke or neurodegenerative illness, cognitive deficits, compulsive behaviour, dementia, depression, Huntington's disease, mania, memory impairment, memory disorders, memory dysfunction, motion disorders, motor disorders, neurodegenerative diseases, Parkinson's disease and Parkinson-like motor disorders, phobias, Pick's disease, psychosis, schizophrenia, spinal cord damage, stroke, and tremor.

In a seventh aspect the invention provides a transgenic animal comprising a knock-out mutation of the endogenous *KCNQ4* gene, a replacement by or an additional expression of a mutated *KCNQ4* gene, or genetically manipulated in order to over-express the *KCNQ4* gene or to over-express mutated *KCNQ4* gene.

In an eighth aspect the invention relates to the use of the transgenic animal of the invention for the *in vivo* screening of therapeutic compounds.

### DETAILED DISCLOSURE OF THE INVENTION

The present invention provides novel voltage-gated potassium channels and genes encoding these channels. The invention also provides cells transformed with these genes, transgenic animals comprising genetic mutations, and the use of the transformed cells and the transgenic animals for the *in vitro* and *in vivo* screening of drugs affecting KCNQ4 containing potassium channels.

### Polynucleotides

In its first aspect, the invention provides novel polynucleotides.

The polynucleotides of the invention are such which have a nucleic acid sequence capable of hybridising under high stringency conditions with the polynucleotide sequence presented as SEQ ID NO: 1, or its complementary strand.

The polynucleotides of the invention include DNA, cDNA and RNA sequences, as well as anti-sense sequences, and include naturally occurring, synthetic, and intentionally manipulated polynucleotides. The polynucleotides of the invention also include sequences that are degenerate as a result of the genetic code.

As defined herein, the term "polynucleotide" refers to a polymeric form of nucleotides of at least 10 bases in length, preferably at least 15 bases in length. By "isolated polynucleotide" is meant a polynucleotide that is not immediately contiguous with both of the coding sequences with which it is immediately contiguous (one on the 5' end and one on the 3' end) in the naturally occurring genome of the organism from which it is derived. The term therefore includes recombinant DNA which is incorporated into an expression vector, into an autonomously replicating plasmid or virus, or into the genomic DNA of a prokaryote or eukaryote, or which exists as a separate molecule, e.g. a cDNA, independent from other sequences.

The polynucleotides of the invention also include allelic variants and "mutated polynucleotides" comprising a nucleotide sequence that differs from the sequence presented as SEQ ID NO: 1 at one or more nucleotide positions. The mutated polynucleotide may in particular be a polynucleotide of the invention having a nucleotide sequence encoding a potassium channel having an amino acid sequence that has been changed at one or more positions located in the conserved regions, as defined by Table 1, below.

In a more specific embodiment the polynucleotide of the invention has the polynucleotide sequence giving rise to the G285S mutation as indicated in SEQ ID NO: 1, i.e. the DNA sequence that at position 935-937 holds the codon AGC rather than the codon GGC stated in SEQ ID NO: 1.

### Hybridisation Protocol

The polynucleotides of the invention are such which have a nucleic acid sequence capable of hybridising with the polynucleotide sequence presented as SEQ ID NO: 1, or its complementary strand, under high stringency conditions, as described in more detail below.

Suitable experimental conditions for determining hybridisation between a nucleotide probe and a homologous DNA or RNA sequence, involves pre-soaking of the filter containing the DNA fragments or RNA to hybridise in 5 x SSC [Sodium chloride/Sodium citrate; cf. *Sambrook et al.;* Molecular Cloning: A Laboratory Manual, Cold Spring Harbor Lab., Cold Spring Harbor, NY 1989] for 10 minutes, and pre-hybridisation of the filter in a solution of 5 x SSC, 5 x Denhardt's solution [cf. *Sambrook et al.; Op cit.*], 0.5 % SDS and 100 µg/ml of denatured sonicated salmon sperm DNA [cf. *Sambrook et al.; Op cit.],* followed by hybridisation in the same solution containing a concentration of 10 ng/ml of a random-primed *[Feinberg A P & Vogelstein B;* Anal. Biochem. 1983 **132** 6-13], ³²P-dCTP-labeled (specific activity > 1 x 10⁹ cpm/µg) probe for 12 hours at approximately 45°C. The filter is then washed twice for 30 minutes in 2 x SSC, 0.5 % SDS at a temperature of at least at least 65°C (medium/high stringency conditions), more preferred of at least 70°C (high stringency conditions), and even more preferred of at least 75°C (very high stringency conditions).

Molecules to which the oligonucleotide probe hybridises under these conditions may be detected using an x-ray film.

### DNA Sequence Homology

In a preferred embodiment, the polynucleotides of the invention show a homology of at least 90%, most preferred at least 95%, with the polynucleotide sequence presented as SEQ ID NO: 1.

As defined herein, the DNA sequence homology may be determined as the degree of identity between two DNA sequences indicating a derivation of the first sequence from the second. The homology may suitably be determined by means of computer programs known in the art such as GAP provided in the GCG program package *[Needleman S B and Wunsch C D,* Journal of Molecular Biology 1970 **48** 443-453] using default parameters suggested herein.

### Cloned Polynucleotides

The isolated polynucleotide of the invention may in particular be a cloned polynucleotide.

As defined herein, the term "cloned polynucleotide", refers to a polynucleotide or DNA sequence cloned in accordance with standard cloning procedures currently used in genetic engineering to relocate a segment of DNA, which may in particular be cDNA, i.e. enzymatically derived from RNA, from its natural location to a different site where it will be reproduced.

Cloning may be accomplished by excision and isolation of the desired DNA segment, insertion of the piece of DNA into the vector molecule and incorporation of the recombinant vector into a cell where multiple copies or clones of the DNA segment will be replicated, by reverse transcription of mRNA (reverse transcriptase technology), and by use of sequence-specific oligonucleotides and DNA polymerase in a polymerase chain reaction (PCR technology).

The cloned polynucleotide of the invention may alternatively be termed "DNA construct" or "isolated DNA sequence", and may in particular be a complementary DNA (cDNA).

It is well established that potassium channels may be formed as heteromeric channels, composed of different subunits. Also it has been found that the potassium channel of the invention may form heteromers with other KCNQ's, in particular KCNQ3, when co-expressed with these subunits. In addition, potassium channels can also associate with non-homologous subunits, e.g. the KCNE1 (formerly known as minK) subunit, that can functionally modulate these channels or lead to a specific localisation within the cell.

Therefore, in a preferred embodiment, the polynucleotide of the invention is cloned and either expressed by itself or co-expressed with polynucleotides encoding other subunits, in particular a polynucleotide encoding a KCNQ3 channel subunit.

### Biological Sources

The isolated polynucleotide of the invention may be obtained from any suitable source. In a preferred embodiment, which the polynucleotide of the invention is cloned from, or produced on the basis of a cDNA library, e.g. of the retina, brain, skeletal muscle. Commercial cDNA libraries are available from e.g. Stratagene and Clontech.

The isolated polynucleotide of the invention may be obtained by methods known in the art, e.g. those described in the working examples below.

In a preferred embodiment the polynucleotide of the invention may be obtained using the PCR primers described in the working examples and presented as SEQ ID NOS: 3-32.

### Preferred Polynucleotides

In a preferred embodiment, polynucleotide of the invention comprises the polynucleotide sequence presented as SEQ ID NO: 1.

In another preferred embodiment the polynucleotide of the invention is a sequence giving rise to KCNQ4 channels subunits comprising one or more substitutions.

In yet another preferred embodiment the polynucleotide of the invention is a sequence giving rise to KCNQ4 channels subunits comprising one or more substitutions in the conserved regions, as defined in Table 1 below.

In a more preferred embodiment the polynucleotide of the invention has a polynucleotide sequence giving rise to the G285S mutation as indicated in SEQ ID NO: 1, e.g. the DNA sequence that at position 935-937 holds the codon AGC rather than the codon GGC stated in SEQ ID NO: 1.

Also contemplated within the scope of this invention are the primer sequences used in Example 2 below for the amplification of the single KCNQ4 exons, that can then be screened for mutations.

It has been demonstrated that KCNQ channels often show alternative splicing and therefore may occur as isoforms originating from the same gene. Such isoforms as well as the different cDNA sequences from which they occurred are also contemplated within the scope of the present invention.

Finally the genes encoding KCNQ channel subunits in other species have been found to differ slightly from the human genes. However, genes of other species, e.g. mouse, rat, monkey, rabbit, etc., are also contemplated within the scope of the present invention.

### Recombinantly Produced Polypeptides

In another aspect the invention relates to substantially pure functional polypeptides that have the electrophysiological and pharmacological properties of a KCNQ4 channel, or KCNQ4 channel subunits. The novel polypeptides of the invention may be obtained by the polynucleotides of the invention using standard recombinant DNA technology.

In a preferred embodiment, a polypeptide of the invention is the KCNQ4 potassium channel subunit comprising the amino acid sequence presented as SEQ ID NO: 2, and biologically active fragments hereof.

Modifications of this primary amino acid sequence may result in proteins which have substantially equivalent activity as compared to the unmodified counterpart polypeptide, and thus may be considered functional analogous of the parent proteins. Such modifications may be deliberate, e.g. as by site-directed mutagenesis, or they may occur spontaneous, and include splice variants, isoforms, homologues from other species, and polymorphisms, and include the variant KCNQ4/G285S (i.e. G285S of SEQ ID NO: 2), that is described in more detail below. Such functional analogous are also contemplated according to the invention.

In the context of this invention, the term "variant polypeptide" means a polypeptide (or protein) having an amino acid sequence that differs from the sequence presented as SEQ ID NO: 2 at one or more amino acid positions. Such variant polypeptides include the modified polypeptides described above, as well as conservative substitutions, splice variants, isoforms, homologues from other species, and polymorphisms, and includes the variant KCNQ4/G285S (i.e. G285S of SEQ ID NO: 2).

As defined herein, the term "conservative substitutions" denotes the replacement of an amino acid residue by another, biologically similar residue. Examples of conservative substitutions include the substitution of one hydrophobic residue such as isoleucine, valine, leucine or methionine for another, or the substitution of one polar residue for another, such as the substitution of arginine for lysine, glutamic for aspartic acid, or glutamine for asparagine, and the like. The term conservative substitution also include the use of a substituted amino acid residue in place of an un-substituted parent amino acid residue provided that antibodies raised to the substituted polypeptide also immunoreact with the un-substituted polypeptide.

Also contemplated within the scope of this invention are the oligopeptides encoded by the primer sequences used in Example 2 below for the amplification of the single KCNQ4 exons, that can then be screened for mutations.

### KCNQ1 Numbering System

In the context of this invention, amino acid residues (as well as nucleic acid bases) are specified using the established one-letter symbol.

By aligning the amino acid sequences of a polypeptide of the present invention to those of the known polypeptides, a specific amino acid numbering system may be employed, by which system it is possible to unambiguously allot an amino acid position number to any amino acid residue in any KNCQ channel protein, which amino acid sequence is known.

Such an alignment is presented in Table 1, below. Using the ClustalX computer alignment program *[Thompson JD, Gibson TJ, Plewniak F, Jeanmougin F, & Higgins DG:* The ClustalX windows interface: flexible strategies for multiple sequence alignment aided by quality analysis tools; Nucleic Acids Res. 1997 **25** (24) 4876-82], and the default parameters suggested herein, the amino acid sequence of a polypeptide of the present invention (hKCNQ4) and the amino acid sequences of the known polypeptides hKCNQ2-3 are aligned with, and relative to, the amino acid sequences of the known polypeptide hKCNQ1 (formerly known as KvLQT1). In the context of this invention this numbering system is designated the KCNQ1 Numbering System.

In describing the various protein variants produced or contemplated according to the invention, the following nomenclatures have been adapted for ease of reference:
*Original amino acid* / *Position* / *Substituted amino acid*

According to this nomenclature the substitution of serine for glycine at position 333 is designated as "G333S".

A deletion of glycine at the same position is designated "G333*".

An insertion of an additional amino acid residue, in this example lysine, may be designated "G333GK" or "*334K" (assumed that no position exists for this position in the amino acid sequence used for establishing the numbering system).

An insertion of an amino acid residue, in this example valine, at a position which exists in the established numbering system, but where no amino acid residue is actually present, may be designated "-301V".

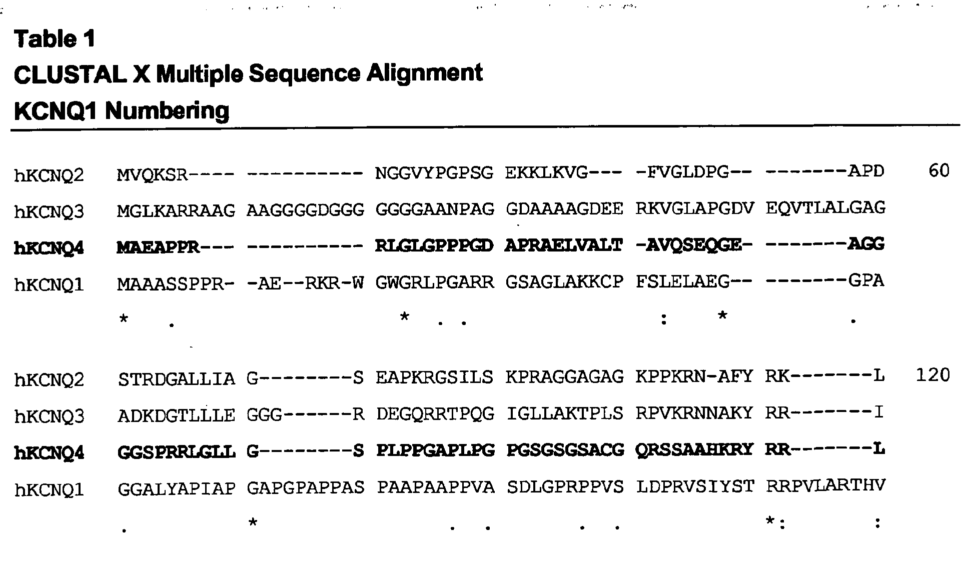

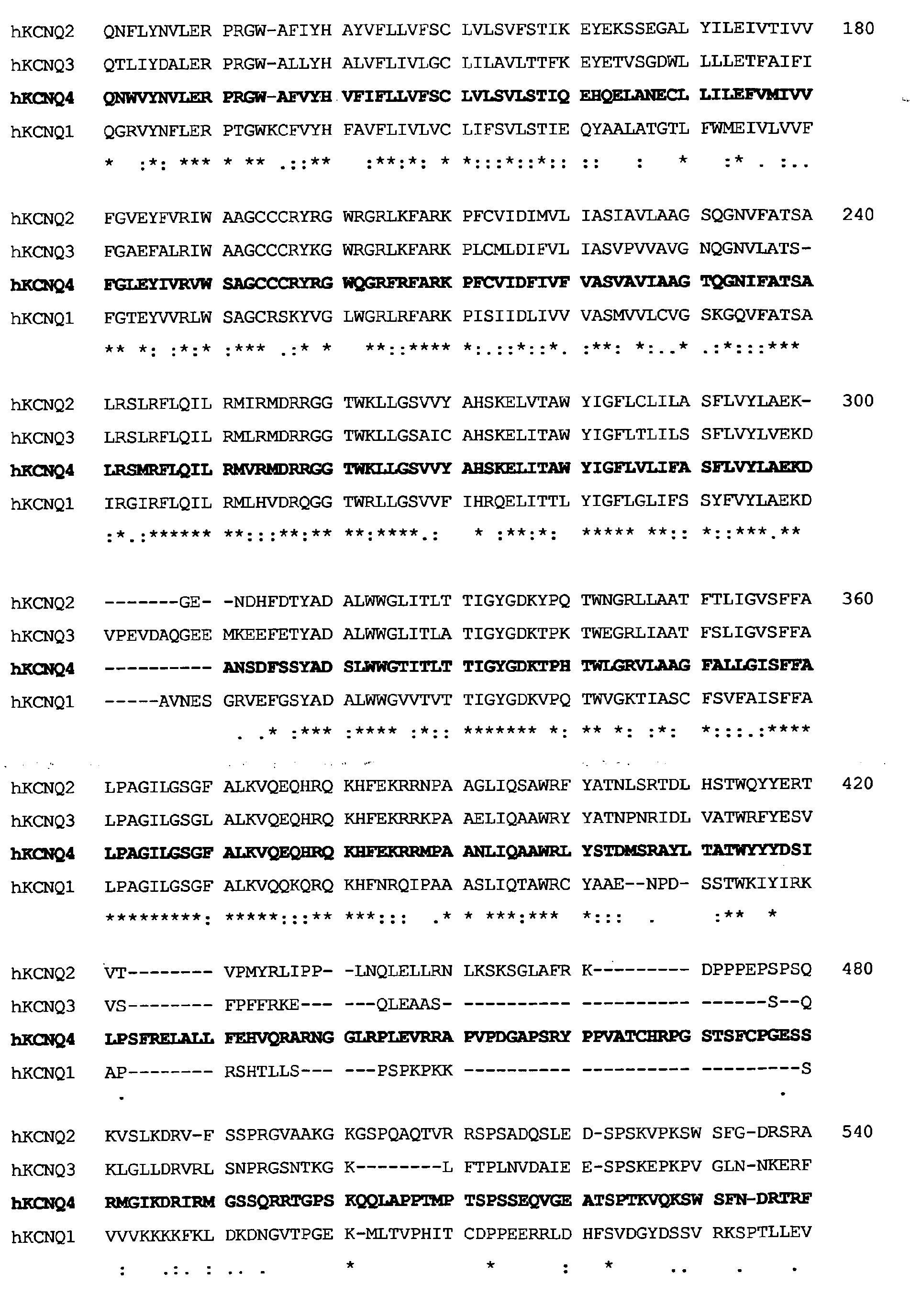

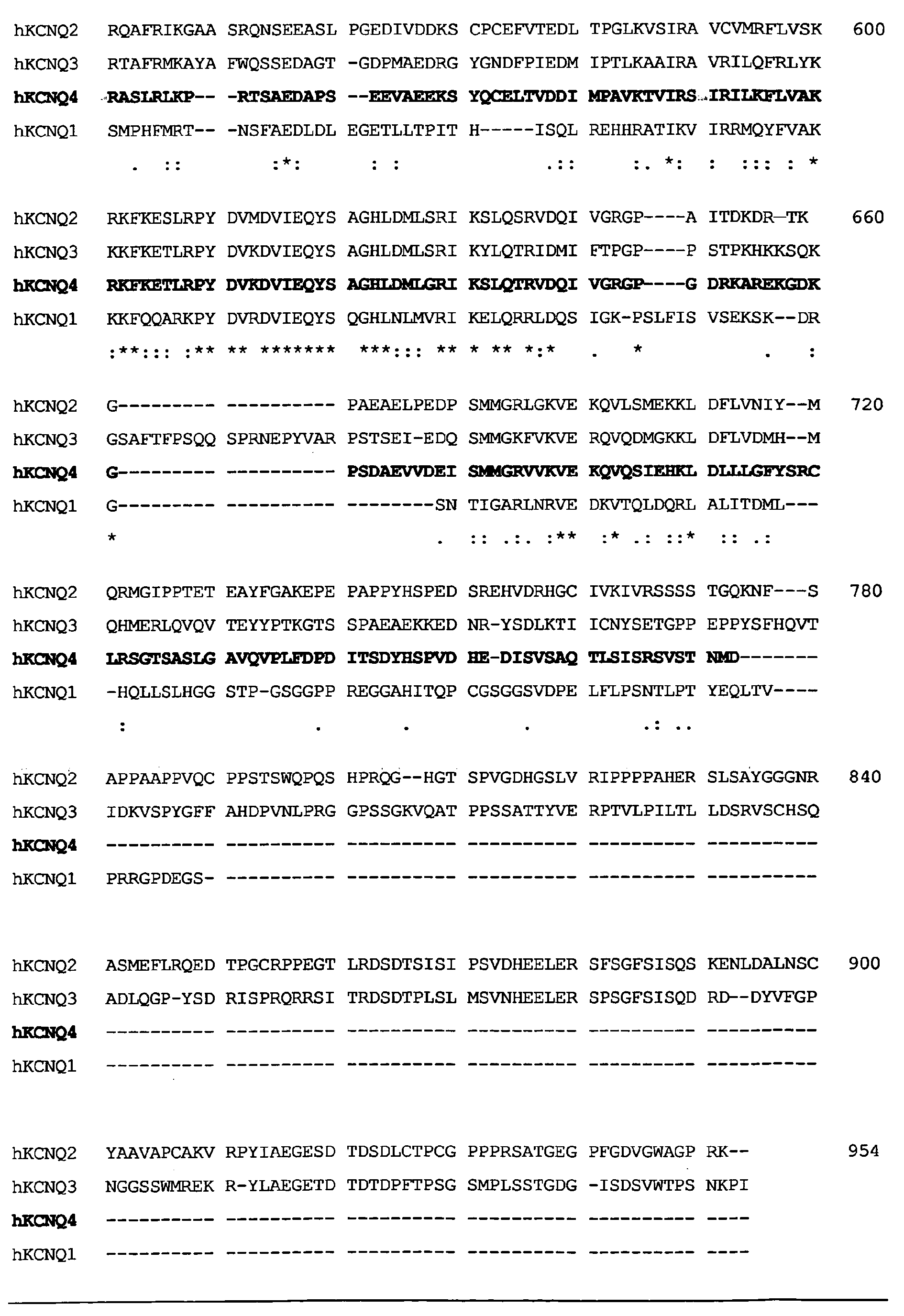

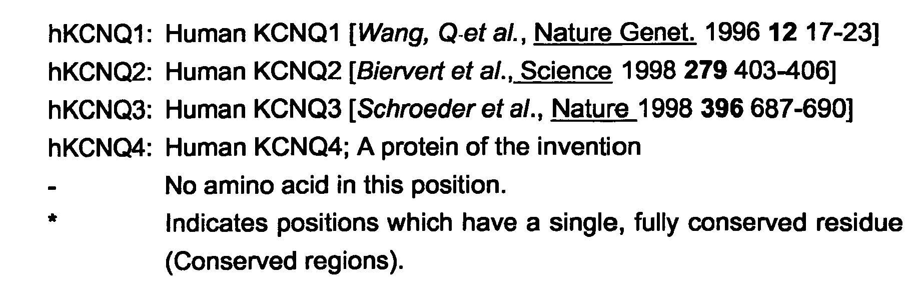

### Biological Activity

The polynucleotide of the invention encodes a potassium channel subunit, which has been termed KNCQ4. In the cochlea, it is differentially expressed in sensory outer hair cells. A mutation in this gene in a pedigree with autosomal dominant hearing loss changes a residue in the KCNQ4 pore region. It abolishes the outwardly rectifying potassium currents of wild-type KCNQ4 on which it exerts a strong dominant negative effect.

Ion channels are excellent targets for drugs. KCNQ4, or heteromeric channels containing the KCNQ4 subunit, may be a particularly interesting target for the treatment of tinnitus and the prevention or treatment of progressive hearing loss.

### KCNQ Channels in Genetic Disease

It is remarkable that mutations in every known *KCNQ* gene lead to human disease: Mutations in *KCNQ1* (KvLQT1) cause the autosomal dominant long QT syndrome (LQTS), and, when present on both alleles, the Jervell and Lange-Nielsen (JLN) syndrome whose symptoms include deafness in addition to cardiac arrhythmias. Mutations in either *KCNQ2* or *KCNQ3,* which form heteromers that probably represent the M-channel, cause benign familial neonatal convulsions (BFNC). The present invention adds *KCNQ4* and the associated autosomal dominant deafness to that list.

After KCNQ1, KCNQ4 is now the second KCNQ channel whose loss of function leads to deafness.

Therefore, in a preferred embodiment of the invention, mutated polynucleotides may be employed in the screening for drugs that affect diseases associated with such mutations in the *KCNQ4* gene.

### Heteromers Formed by KCNQ Subunits

The KCNQ channels described so far function physiologically as heteromers. KCNQ1 associates with KCNE1 (formerly known as minK), and KCNQ2 and KCNQ3 form heteromeric channels that underlie the M-current, an important determinant of neuronal excitability that is regulated by several neurotransmitters.

Like other KCNQ channel subunits, KCNQ4 may interact with other subunits, e.g. KCNE1 or other KCNQ channel subunits, and in particular with KCNQ3. Currents from homomeric KCNQ3 are very small and often cannot be distinguished from *Xenopus* oocyte background currents. Co-expression of KCNQ3 with KCNQ4 markedly increased current amplitudes. Significantly, heteromeric KCNQ3/KCNQ4 channels activated faster than homomeric KCNQ4 channels, the voltage-dependence was shifted to more negative potentials, and currents displayed a different drug sensitivity.

### Antibodies

The polypeptides of the invention can be used to produce antibodies which are immunoreactive or bind to epitopes of these polypeptides. Antibodies which consist essentially of pooled monoclonal antibodies with different specificities, as well as distinct monoclonal antibody preparations may be provided.

The preparation of polyclonal and monoclonal antibodies is well known in the art. Polyclonal antibodies may in particular be obtained as described by e.g. *Green et al.*: "Production of Polyclonal Antisera" in Immunochemical Protocols *(Manson,* Ed.); Humana Press, 1992, Pages 1-5; and *Coligan et al.*: "Production of Polyclonal Antisera in rabbits, rats, Mice and Hamsters" in Current Protocols in Immunology, 1992, Section 2.4.1; which protocols are hereby incorporated by reference.

Monoclonal antibodies may in particular be obtained as described by e.g. *Kohler & Milstein*, Nature 1975 **256** 495; Coligan et al. in Current Protocols in Immunology, 1992, Sections 2.5.1 - 2.6.7; and *Harlow et al.* in Antibodies: A Laboratory Manual; Cold Spring Harbor Pub., 1988, Page 726; which protocols are hereby incorporated by reference.

Briefly, monoclonal antibodies may be obtained by injecting e.g. mice with a composition comprising an antigen, verifying the presence of antibody production by removing a serum sample, removing the spleen to obtain B lymphocytes, fusing the B lymphocytes with myeloma cells to produce hybridomas, cloning the hybridomas, selecting positive clones that produce the antibodies to the antigen, and isolating the antibodies from the hybridoma cultures.

Monoclonal antibodies can be isolated and purified from hybridoma cultures by a variety of well-established techniques, including affinity chromatography with protein A Sepharose, size-exclusion chromatography, and ion-exchange chromatography, see. e.g. *Coligan et al.* in Current Protocols in Immunology, 1992, Sections 2.7.1 - 2.7.12, and Sections 2.9.1 - 2.9.3; and *Barnes et al.*: "Purification of Immunoglobulin G (IgG)" in Methods in Molecular Biology; Humana Press, 1992, Vol. 10, Pages 79-104.

The polyclonal or monoclonal antibodies may optionally be further purified, e.g. by binding to and elution from a matrix to which the polypeptide, to which the antibodies were raised, is bound.

Antibodies which bind to the polypeptide of the invention can be prepared using an intact polypeptide or fragments containing small peptides of interest as the immunising antigen. The polypeptide used to immunise an animal may be obtained by recombinant DNA techniques or by chemical synthesis, and may optionally be conjugated to a carrier protein. Commonly used carrier proteins which are chemically coupled to the peptide include keyhole limpet hemocyanin (KLH), thyroglobulin, bovine serum albumin (BSA), and tetanus toxoid. The coupled peptide may then be used to immunise the animal, which may in particular be a mouse, a rat, a hamster or a rabbit.

### Genetically Manipulated Cells

In a third aspect the invention provides a cell genetically manipulated by the incorporation of the heterologous polynucleotide of the invention. The cell of the invention may in particular be genetically manipulated to transiently or stably express, over-express or co-express a KCNQ4 channel subunit as defined above. Methods of transient and stable transfer are known in the art.

The polynucleotide of the invention may be inserted into an expression vector, e.g. a plasmid, virus or other expression vehicle, and operatively linked to expression control sequences by ligation in a way that expression of the coding sequence is achieved under conditions compatible with the expression control sequences. Suitable expression control sequences include promoters, enhancers, transcription terminators, start codons, splicing signals for introns, and stop codons, all maintained in the correct reading frame of the polynucleotide of the invention so as to permit proper translation of mRNA. Expression control sequences may also include additional components such as leader sequences and fusion partner sequences.

The promoter may in particular be a constitutive or an inducible promoter. When cloning in bacterial systems, inducible promoters such as pL of bacteriophage γ, plac, ptrp, ptac (ptrp-lac hybrid promoter), may be used. When cloning in mammalian systems, promoters derived from the genome of mammalian cells, e.g. the TK promoter or the metallothionein promoter, or from mammalian viruses, e.g. the retrovirus long terminal repeat, the adenovirus late promoter or the vaccinia virus 7.5K promoter, may be used. Promoters obtained by recombinant DNA or synthetic techniques may also be used to provide for transcription of the polynucleotide of the invention.

Suitable expression vectors typically comprise an origin of expression, a promoter as well as specific genes which allow for phenotypic selection of the transformed cells, and include vectors like the T7-based expression vector for expression in bacteria *[Rosenberg et al;* Gene 1987 56 125], the pMSXND expression vector for expression in mammalian cells [*Lee and Nathans,* J. Biol. Chem. 1988 **263** 3521], baculovirus derived vectors for expression in insect cells, and the oocyte expression vector PTLN *[Lorenz C, Pusch M & Jentsch T* J: Heteromultimeric CLC chloride channels with novel properties; Proc. Natl. Acad. Sci. USA 1996 **93** 13362-13366].

In a preferred embodiment, the cell of the invention is a eukaryotic cell, in particular a mammalian cell, an oocyte, or a yeast cell. In a more preferred embodiment, the cell of the invention is a human embryonic kidney (HEK) cell, a HEK 293 cell, a BHK21 cell, a Chinese hamster ovary (CHO) cell, a *Xenopus laevis* oocyte (XLO) cell, a COS cell, or any other cell line able to express KCNQ potassium channels.

When the cell of the invention is a eukaryotic cell, incorporation of the heterologous polynucleotide of the invention may be in particular be carried out by infection (employing a virus vector), by transfection (employing a plasmid vector), or by calcium phosphate precipitation, microinjection, electroporation, lipofection, or other physical-chemical methods known in the art.

In a further preferred embodiment, the cell of the invention is genetically manipulated to co-express the KCNQ4 channel subunit of the invention and a KCNQ1 channel subunit; the KCNQ4 channel subunit of the invention and a KCNQ2 channel subunit; the KCNQ4 channel subunit of the invention and a KCNQ3 channel subunit; the KCNQ4 channel subunit of the invention and KCNQ1 and KCNQ2 channel subunits; the KCNQ4 channel subunit of the invention and KCNQ1 and KCNQ3 channel subunits; the KCNQ4 channel subunit of the invention and KCNQ2 and KCNQ3 channel subunits; or the KCNQ4 channel subunit of the invention and KCNQ1 and KCNQ2 and KCNQ3 channel subunits.

### Screening of Drugs

In a further aspect the invention provides methods for screening for chemical compounds capable of binding to, and showing activity at potassium channels containing one or more KCNQ4 subunits. The activity determined may be inhibitory activity, stimulating activity, or other modulatory activity. In particular the KCNQ4 active compound may induce a second messenger response, which cause a change of the molecular characteristics of the cell, e.g. the ion flux, enzyme activation, changes in cyclic nucleotides such as cAMP, cADP, cGMP, and cGDP, etc.

Therefore, in another aspect, the invention provides a method for identifying functional ligands for a human potassium channel, comprising a KCNQ4 subunit of the invention, which method comprises transfecting cells with one or more polynucleotides of the invention, encoding a KCNQ4 channel subunit, and detecting the effect on the signal transduction pathway caused in these cells by binding of the ligands to the receptor by a reporter system.

Such chemical compounds can be identified by one of, or both methods described below.

### Binding Studies

Binding studies are usually carried out by subjecting the target to binding with a labelled, selective agonist (binding agent), to form a labelled complex, followed by determination of the degree of displacement caused by the test compound upon addition to the complex.

In a specific aspect the invention provides a method of screening a chemical compound for capability of binding to a potassium channel comprising at least one KCNQ4 channel subunit, which method comprises the steps of (i) subjecting a KCNQ4 channel subunit containing cell of the invention to the action of a an agent capable of binding to the KCNQ4 polypeptide to form a complex with the KCNQ4 channel subunit containing cell; (ii) subjecting the complex of step (i) to the action of the chemical compound to be tested; and (iii) detecting the displacement of the KCNQ4 binding agent from the complex with the KCNQ4 channel subunit containing cell.

The KCNQ4 binding agent is a radioactively labelled 1,3-dihydro-1-phenyl-3,3-bis(4-pyridylmethyl)-2H-indol-2-one (Linopirdine); or 10,10-bis(4-pyridinyl-methyl)-9(1 OH)-antracenone.

In a preferred embodiment, the biding agent is labelled with ³H, and the displacement of the KCNQ4 binding agent from the complex with the KCNQ4 channel subunit containing cell is detected by measuring the amount of radioactivity by conventional liquid scintillation counting.

### Activity Studies

The KCNQ4 channel agonists may affect the potassium channel in various ways. The agonist may in particular show inhibitory activity, stimulating activity, or other modulatory activity.

In a specific aspect the invention provides a method for determining the activity at potassium channels containing one or more KCNQ4 subunits. According to this method a KCNQ4 channel subunit containing cell of the invention is subjecting to the action of the chemical compound to be tested, and the activity is detected by way of monitoring the membrane potential, the current, the potassium flux, or the secondary calcium influx of the KCNQ4 channel subunit containing cell, preferably a genetically manipulated as described above.

The membrane potential and the current may be monitored by electrophysiologic methods, including patch clamp techniques, such as current clamp technology and two-electrode voltage clamp technology, or by spectroscopic methods, such as fluorescence methods.

In a preferred embodiment, monitoring of the membrane potential of the KCNQ4 channel subunit containing cell is performed by patch clamp techniques.

In another preferred embodiment, monitoring of the membrane potential of the KCNQ4 channel subunit containing cell is performed by spectroscopic methods, e.g. using fluorescence methods. In a more specific embodiment, the KCNQ4 channel subunit containing cell is mixed with a membrane potential indicating agent, that allow for a determination of changes in the membrane potential of the cell, caused by the addition of the test compound. The membrane potential indicating agent may in particular be a fluorescent indicator, preferably DIBAC₄(3), DiOC5(3), and DiOC2(3).

In yet a preferred embodiment, monitoring of the membrane potential of the KCNQ4 channel subunit containing cell is performed by spectroscopic methods, e.g. using a FLIPR assay (Fluorescence Image Plate Reader; available from Molecular Devices).

### Screening of Genetic Material

In a further aspect the invention relates to the use of a polynucleotide sequence of the invention for the screening of genetic materials. By this method, individuals bearing a gene identical or homologous to a polynucleotide of the invention may be identified.

In the screening method of the invention, a polynucleotide of the invention, or any fragment or sub-sequence hereof, and in particular a polynucleotide sequence holding the mutation giving rise to the KCNQ4/G285S variant (i.e. G285S of SEQ ID NO: 2) is employed.

In the screening method of the invention only short sequences needs to be employed depending on the actual method used. For SSCA, several hundreds of base pairs may be needed, for oligonucleotide or PCR hybridisation only of from about 10 to about 50 basepairs may be needed.

The screening may be accomplished by conventional methods, including hybridisation, SSCA analysis, and array technology (DNA chip technology). The hybridisation protocol described above represents a suitable protocol for use in a screening method of the invention.

In a preferred embodiment, the method is used for performing gene amplification using conventional PCR techniques, e.g. as described in the working examples below.

### Transgenic Animals

Transgenic animal models provide the means, *in vivo,* to screen for therapeutic compounds. The establishment of transgenic animals may in particular be helpful for the screening of drugs to fully elucidate the pathophysiology of *KCNQ4*/DFNA2 deafness. These animals may also be valuable as a model for the frequent condition of presbyacusis that also develops slowly over decades. Since KCNQ4 is expressed also in brain, they may also be helpful in screening for drugs effective in CNS disorders, e.g. epilepsy.

By transgene is meant any piece of polynucleotide which is inserted by artifice into a cell, and thus becomes part of the genome of the organism that develops from that cell. Such a transgene may include a gene which is partly or entirely heterologous (i.e. foreign) to the transgenic organism, or it may represent a gene homologous to an endogenous gene of the organism.

By a transgenic animal is meant any organism holding a cell which includes a polynucleotide sequence which is inserted into that cell by artifice, and which cell becomes part of the transgenic organism which develops from that cell. Such a transgene may be partly or entirely heterologous to the transgenic animal. Although transgenic mice represent a preferred embodiment of the invention, other transgenic mammals including, but not limited to transgenic rodents (e.g., hamsters, guinea pigs, rabbits and rats), and transgenic pigs, cattle, sheep and goats may be created by standard techniques and are included in the invention.

Preferably, the transgene is inserted by artifice into the nuclear genome.

### Knock-out and Knock-in Animals

The transgenic knock-out animal models may be developed by homologous recombination of embryonic stem cells with constructs containing genomic sequence from the *KCNQ4* gene, that lead to a loss of function of the gene after insertion into the endogenous gene.

By knock-out mutation is meant an alteration in the polynucleotide sequence that reduces the biological activity of the polypeptide normally encoded therefrom. In order to create a true knock-out model, the biological activity of the expressed polypeptide should be reduced by at least 80% relative to the un-mutated gene. The mutation may in particular be a substitution, an insertion, a deletion, a frameshift mutation, or a mis-sense mutation. Preferably the mutation is a substitution, an insertion or a deletion.

To further assess the role of KCNQ4 at an organism level, the generation of an animal, preferably a mouse, lacking the intact KCNQ4 gene, or bearing a mutated *KCNQ4* gene, is desired.

A replacement-type targeting vector, which may be used to create a knock- out model, may be constructed using an isogenic genomic clone, e.g. from a mouse strain such as 129/Sv (Stratagene Inc., La Jolla, CA). The targeting vector may be introduced into a suitably-derived line of embryonic stem (ES) cells by electroporation to generate ES cell lines that carry a profoundly truncated form of the *KCNQ4* gene. The targeted cell lines may then be injected into a mouse blastula stage embryo to generate chimeric founder mice. Heterozygous offspring may be interbred to homozygosity.

As the slowly progressive hearing loss observed in DFNA2 may require the expression from one allele of a dominant negative mutant, it may also be desired to create a knock-in animal in which the wild-type KCNQ4 gene is replaced by this mutated gene.

Animal models for over-expression may be generated by integrating one or more polynucleotide sequence of the invention into the genome according to standard techniques.

The procedures disclosed herein involving the molecular manipulation of nucleic acids are known to those skilled in the art, and are described by e.g. *Fredrick MA et al.* [*Fredrick MA et al.:* Short Protocols in Molecular Biology; John Wiley and Sons, 1995] and *Sambrook et al. [Sambrook et al.:* Molecular Cloning: A Laboratory Manual; 2. Ed.,. Cold Spring Harbor Lab.; Cold Spring Harbor, NY 1989], and in *Alexandra LJ* (Ed.): Gene Targeting: A practical approach; Oxford University Press (Oxford, New York, Tokyo), 1993.

### BRIEF DESCRIPTION OF THE DRAWINGS

The present invention is further illustrated by reference to the accompanying drawing, in which:
Fig. 1 shows the electrophysiological properties of KCNQ4 currents:
   Fig. 1A: Two-electrode voltage-clamp current traces from a *Xenopus* oocyte injected with KCNQ4 cRNA. Starting from a holding potential of -60 mV cells were clamped for 4 seconds to voltages between -80...+60 mV in +10 mV steps, followed by a constant test pulse to -30 mV;
   Fig. 1B: Current traces showing the inactivation behaviour of KCNQ4 at different voltages. After an activating voltage pulse at +40 mV of 3.5 seconds duration the cell was clamped to voltages between +40...-120 mV in -10 mV steps;
   Fig. 1C: Apparent open-probability (pₒₚₑₙ) as a function of voltage determined from tail current analysis of currents as in (A). Half-maximal pₒₚₑₙ is archived at (-10.0±1.2) mV, and the apparent gating charge is 1.4±0.1, as obtained from a fit of a Boltzmann-function to the data (n=14 from 2 oocyte batches, ±S.E.M.);

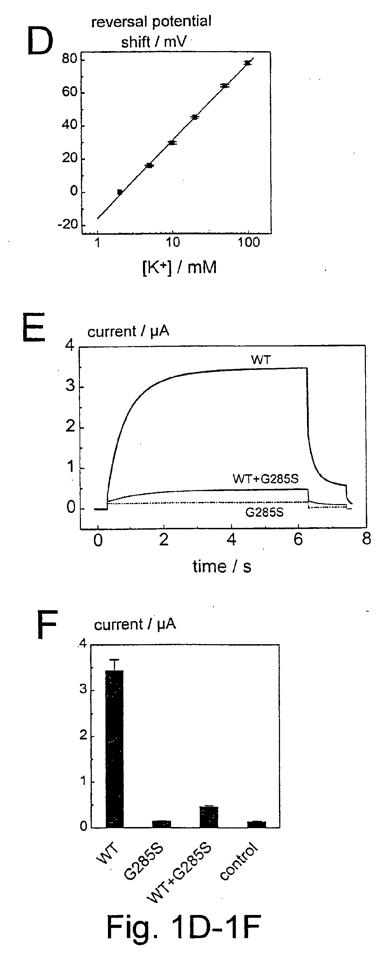

   Fig. 1D: Shift of the reversal potential with the extracellular K⁺⁻concentration with ND98 as reference solution. Total mono -valent cation concentration was 100 mM and the stated K⁺-concentration was obtained by mixing solutions ND100 and KD100. The reversal potential shift of 46.7±0.9 mV per decade indicates a channel selective for K⁺ (n=18 from 3 oocyte batches, ±S.E.M.). Substitution of external K⁺ with other cations yielded the following permeability ratios: P_{K}/P_{Na} = 52.3±4.4, P_{K}/P_{Cs} = 7.8±0.7, and P_{K}/P_{Rb} = 0.94±0.03 (permeability sequence: Rb^{+ ~}K⁺ > Cs⁺ >> Na⁺, n=15 from 3 oocyte batches, ±S.E.M.);
   Fig. 1E: Current traces of WT KCNQ4 (thick solid line), a 1: 1 co-injection of WT KCNQ4 and KCNQ4_{G285S} mutant (thin solid line) and KCNQ4_{G285S} mutant (dotted line). KCNQ4_{G285S} currents were indistinguishable from water-injected control oocytes. From a holding potential at -60 mV the cells were voltage-clamped for 6 seconds at +40 mV, followed by a -30 mV step;
   Fig. 1F: Mean currents, measured after clamping oocytes for 4 seconds at +40 mV, averaged from several experiments as in (1 E) (n=20...35, 4 oocyte batches, ±S.E.M.); and
   Fig. 2A shows KCNQ4 co-expressed with KCNQ1;
   Fig. 2B shows KCNQ4 co-expressed with KCNQ2;
   Fig. 2C shows KCNQ4 co-expressed with KCNQ3;
   Fig. 2D shows derived dominant negative mutants (KCNQ1_{G219S}, KCNQ2_{G279S}, KCNQ3_{G318S}) (n=10...31, 3 oocyte batches, ±S.E.) Representative currents from experiments as in (C) showing altered activation kinetics for the co-injection of KCNQ4 with KCNQ3 or KCNQ3_{G318S}, respectively. From a holding potential at -60 mV the voltage was clamped for 4 s at +40 mV, followed by a step to -30 mV. Time constants and amplitudes obtained from two-exponential fits were: KCNQ4: t₁=360 ms, A₁=-4.9 µA, t₂=1700 ms, A₂=-0.34 µA; KCNQ3+KCNQ4: t₁=120 ms, A₁=-6.3 µA, t₂=560ms, A₂=-1.3 µA;
   Fig. 2E: Apparent pₒₚₑₙ as a function of voltage for currents from oocytes co-injected with KCNQ4 and KCNQ3 cRNA, determined from tail current analysis (squares, thick solid curve). Half-maximal pₒₚₑₙ is achieved at V_{0.5} = (-19.1 ± 2.0) mV, and the apparent gating charge is 1.5±0.2 (n=23 from 3 oocyte batches, ±S.E.M.), as obtained from a fit of a Boltzmann-function to the data. The pₒₚₑₙ curve for KCNQ4 is also shown for reference (circles, thin solid curve);
   Fig. 2F: Current traces recorded from an oocyte co-injected with KCNQ3 and KCNQ4 cRNA with typical M-current voltage-protocol. Starting from a holding potential at -30 mV the cell was progressively hyperpolarized for 1 s to voltages between -30 and -90 mV in -10 mV steps;
   Fig. 2G: Differential effects of 200 µM Linopirdine on KCNQ4 (n=10, ±S.E.M.) and KCNQ3+KCNQ4 (n=6, ±S.E.M.). Currents were measured at +40 mV and % current remaining with Linopirdine is shown. Upon addition of Linopirdine steady-state inhibition was reached after ~1 min for KCNQ4 currents, and after -3 min for KCNQ3+KCNQ4 currents.

### EXAMPLES

The invention is further illustrated with reference to the following examples which are not intended to be in any way limiting to the scope of the invention as claimed.

### Example 1

### Cloning and Characterisation of KCNQ4 cDNA

Using a KCNQ3 potassium channel partial cDNA as a probe, a human retina cDNA λ phage library (Clontech, #HL1132a) was screened, and a ≈1 kb cDNA encoding a protein fragment homologous to KCNQ potassium channels was isolated. It was distinct from the known members KCNQ1 (KvLQT1), KCNQ2 and KCNQ3. We named the novel gene *KCNQ4.* Overlapping cDNA's containing the entire open reading frame were obtained by re-screening the cDNA library and by extending the 5' end in RACE (rapid amplification of cDNA ends) experiments using a Marathon kit (Clontech) with human skeletal muscle cDNA. A complete cDNA was assembled and cloned into the oocyte expression vector PTLN *[Lorenz C, Pusch M & Jentsch T J:* Heteromultimeric CLC chloride channels with novel properties; Proc. Natl. Acad. Sci. USA 1996 **93** 13362-13366].

The cDNA encodes a polypeptide of 695 amino acids with a predicted mass of 77 kDa (SEQ ID NO: 2). Its overall amino-acid identity to KCNQ1, KCNQ2, and KCNQ3 is 38%, 44%, and 37%, respectively. Together with these proteins it forms a distinct branch of the superfamily of voltage-gated potassium channels. As a typical member of this gene family, KCNQ4 has 6 predicted transmembrane domains and a P-loop between transmembrane domains S5 and S6. In potassium channels, which are tetramers of identical or homologous subunits, four of these highly conserved P-loops combine to form the ion-selective pore. As other KCNQ channels, KCNQ4 has a long predicted cytoplasmic carboxy terminus that accounts for about half of the protein. A conserved region present in the carboxy termini of KCNQ1, -2, and -3 is also present in KCNQ4 (roughly represented by exon 12).

The sequence of KCNQ4 predicts several potential sites for phosphorylation by protein kinase C. In contrast to KCNQ1 and KCNQ2, however, it lacks an amino terminal consensus site for cAMP-dependent phosphorylation.

A human multiple tissue Northern blot (Clontech, #7760-1) was probed with a 749 bp EcoRI/Pmll cDNA fragment of KCNQ4. The fragment was labelled with ³²P using the Rediprime labelling kit (Amersham). Hybridisation was performed in ExpressHyb solution according to the instructions of the manufacturer (Clontech). The filter was then exposed to Kodak BioMax film for 4 days.

Northern analysis of *KCNQ4* expression in human tissues revealed faint bands of ≈5 kb in heart, brain and skeletal muscle. In some tissues, there was also a larger band. Upon longer exposure, weaker ≈5 kb bands were also detected in other tissues including kidney and pancreas.

### Example 2

### Genomic structure and chromosomal mapping to the DFNA2 locus

A PAC was isolated that contains the entire *KCNQ4* coding region. The genomic structure of the *KCNQ4* gene was established (SEQ ID NO:1).

The genomic structure was established by a PCR approach from genomic DNA. Individual *KCNQ4* exons and adjacent short intronic sequences were amplified by standard PCR techniques from human genomic DNA using intronic oligonucleotide primers.

For amplification, the following intronic primer pairs were used (all primers in 5'→3' direction; in brackets the size of the PCR product):
1a (SEQ ID NO: 3): catgcgtctctgagcgccccgagc
   1r (SEQ ID NO: 4): aggccaggcttgcgcggggaaacg (544)
2a (SEQ ID NO: 5): cagcacagagctgtaactccagg
   2r (SEQ ID NO: 6): aagctgctctctgagccatgg (500)
3a (SEQ ID NO: 7): gctgggtccgcgctgtgacc
   3r (SEQ ID NO: 8): ggtctccagggtcagagtcg (292)
4a (SEQ ID NO: 9): tccgggtccgtgcgcggggta
   4r (SEQ ID NO: 10): gagacagcccctctgacctcg (328)
5a (SEQ ID NO: 11): atccctttcccgtgtggaagc
   5r (SEQ ID NO: 12): agtcacgatgggcagacctcg (286)
6a (SEQ ID NO: 13): cctcatgatcaggctcctacc
   6r (SEQ ID NO: 14): atgtgtgacaggggtgagc (270)
7a (SEQ ID NO: 15): aaggatggggacacccttgc.
   7r (SEQ ID NO: 16): acacagggttgacacacc (244)
8a (SEQ ID NO: 17): gctctgggtaacccacaactg
   8r (SEQ ID NO: 18): gctcccctgggagccatcacc (316)
9a (SEQ ID NO: 19): tgagctcaggagctctgtgc
   9r (SEQ ID NO: 20): acccacgaagtggctgaaggc (346)
10a (SEQ ID NO: 21): gtcctaagtcagctttgtcc
   10r (SEQ ID NO: 22): cctcagccggccctcgatcg (347)
11a (SEQ ID NO: 23): cactctactggtggtttggc
   11r (SEQ ID NO: 24): ctcctgacctcaagtgatcc (281)
12a (SEQ ID NO: 25): gatagcaaagagatggagagg
   12r (SEQ ID NO: 26): aactcagctgcagcagtgagc (328)
13a (SEQ ID NO: 27): gtgccttctccttcatcaggc
   13r (SEQ ID NO: 28): aacgcatcctccccatgtca (297)
14a (SEQ ID NO: 29): tttgtgcttcccagataagc
   14r (SEQ ID NO: 30): cgtgagggagtgagttcaagtacg (445)

Sequences of exons and adjacent introns are deposited in GenBank (Accession Numbers AF105203-AF105216).

To screen unlinked pedigrees with autosomal dominant deafness we amplified only exons 4 through 7 that code for the pore and adjacent transmembrane domains as these may have the highest likelihood to harbour mutations. After amplification and agarose gel purification, PCR products were directly sequenced using the amplification primers and an ABI377 automated DNA sequencer.

The highly conserved transmembrane block S1-S6 was found to be encoded by 6 exons (exons 2 to 7) having the same limits as in KCNQ2 and KCNQ3. In KCNQ1 an additional intron interrupts the sequence encoding domain S4. The exon-intron structures of KCNQ genes diverge most in the poorly conserved carboxy-termini of these proteins.

Using hybridisation to human chromosomes, *KCNQ4* was mapped to 1 p34.

A PAC containing the coding sequence of *KCNQ4* was isolated using intronic KCNQ4 oligonucleotide primers and PCR. It was used to localise *KCNQ4* to 1 p34 using FISH (Genome Systems). KCNQ4 was then mapped on the Whitehead Contig WC1.10 using several of the intronic primers given above and published STS markers by PCR amplification from individual YAC clones.

Several diseases have been mapped to this region. This includes DFNA2, a locus for dominant progressive hearing loss. Due to the critical role of K⁺ homeostasis in auditory mechano transduction, we considered *KCNQ4* as an excellent candidate gene for DFNA2. The DFNA2 locus has been mapped between markers D1S255 and D1S193. We therefore refined the localisation of *KCNQ4* in comparison to published physical and genetic maps using a YAC (yeast artificial chromosome) contig of this region. *KCNQ4* was present on CEPH YAC clone 914c3, a result which places this gene within the DFNA2 region.

### Example 3

### Expression of KCNQ genes in the inner ear

The expression of *KCNQ4,* as well as of other KCNQ genes, was studied by semi-quantitative RT-PCR on mouse cochlear RNA.

### RT-PCR analysis of mouse KCNQ mRNA expression

Approximately 2 µg of mouse total brain RNA and mouse cochlear and vestibular RNA were reverse transcribed using the SuperScript^{™} II (Gibco BRL) reverse transcriptase.

1 µl (resp. 1 µl of a 1:10 dilution) of cDNA was amplified for 30 cycles (96°C for 30 sec, 61°C for 30 sec, and 68°C for 45 sec) using a 2400 Thermocycler System (Perkin Elmer). Each 50 µl reaction contained 2.5 U polymerase (Expand^{™} Long Template PCR System, Boehringer Mannheim) and 5% DMSO.

*KCNQ1* primers were based on the mouse cDNA sequence (GenBank Accession # U70068):
MK1a 5'-aaggctggatcagtccattgg-3'; and
   MK1 r 5'-aggtgggcaggctgttgctgg-3' (280 bp)

As no mouse KCNQ2 sequence was available, we chose sequences conserved between human (Y15065) and rat (AF087453) KCNQ2:
MK2a 5'-gccacggcacctcccccgtgg-3'; and
   MK2r 5'-ccctctgcaatgtagggcctgac-3' (331 bp)
KCNQ3 primers were derived from a mouse EST (AA386747):
MK3a 5'-ccaaggaatgaaccatatgtagcc-3'; and
   MK3r 5'-cagaagagtcaagatgggcaggac-3' (461 bp)
Mouse *KCNQ4* primers were:
(SEQ ID NO: 31): MK4a 5'-agtacctgatggagcgccctctcg-3'; and
   (SEQ ID NO: 32): MK4r 5'-tcatccaccgtaagctcacactgg-3' (366 bp)

Amplification products were verified by direct sequencing.

These results were compared with those obtained with vestibular and brain RNA.

*KCNQ1, KCNQ3* and *KCNQ4* messages can be detected in the cochlea, and additional PCR cycles revealed a weak KCNQ2 expression as well. At this high amplification, KCNQ1 was also detected in brain. *KCNQ1* and *KCNQ4* appear to have the highest cochlear expression. *KCNQ1* expression is higher in the cochlea than in brain (which was negative by Northern analysis). The reverse is true for *KCNQ2* and *KCNQ3,* both of which are broadly expressed in brain. *KCNQ4* expression is significant in both of these tissues.

### In situ hybridisation of mouse cochlea

*In situ* hybridisation's were performed on cochlea sections from mice at postnatal day P12 with a KCNQ4 antisense probe.

A mouse *KCNQ4* cDNA corresponding to bp 618 to 1602 of the human *KCNQ4* ORF was cloned into pBluescript. Sense and antisense probes were transcribed using T3 and T7 RNA polymerases after appropriate linearization. After DNAse digestion, the probes were ethanol precipitated twice with 0.4 M LiCl. They were labelled with digoxigenin-11-UTP as described previously (Schaeren-Wiemers and Gerfin-Moser, 1993).

Mouse inner ears were fixed for 1 hour at 4 °C in 4 % paraformaldehyde in PBS. After three rinses in PBS, they were immersed in 20 % sucrose overnight at 4°C. Cryostat sections (10-14 µm) were post-fixed and rinsed in PBS. Following pre-hybridisation at room temperature for at least 3 hours, they were hybridised overnight at 58 °C in a humid chamber. Sections were then washed and incubated with sheep antidigoxigenin antibody coupled to alkaline phosphatase. Staining by NBT/BCIP (Boehringer Mannheim) was done for 2 hours at 37°C and overnight at RT. Sections were then mounted in Aquatex (Merck, USA).

Sensory outer hair cells were strongly labelled. By contrast, the inner hair cells appeared negative. The stria vascularis, the site of *KCNQ1* expression, was negative as well. Control hybridisation with a *KCNQ4* sense probe revealed that the staining of outer hair cells was specific.

### Autosomal Dominant Deafness

These results indicated that *KCNQ4* was an excellent candidate gene for autosomal dominant deafness. As we did not have access to the published pedigrees that were linked to the DFNA2 locus, we screened 45 families with autosomal dominant deafness without previous linkage analysis. In most of these families, the hearing loss had been diagnosed before adulthood, i.e. before the age of onset reported for most of the DFNA forms, including DFNA2.

Mutation screening was limited to exons 4 to 7 that encode the pore region and adjacent transmembrane domains. A *KCNQ4* mutation was found in a French family with profound hearing loss. Its clinical features include progressive hearing loss that is more prominent with higher frequencies, tinnitus in one patient, and no indication for vestibular defects nor gross morphological changes in the inner ear. A mis-sense mutation (cf. SEQ ID NO: 1; The mutation G935A at the nucleotide level giving rise to the variant G285S at the amino acid level) was present in exon 6 in a heterozygous state. Using an Alul restriction site (AGCT) introduced by this mutation, it was shown that it co-segregated with all affected members in the pedigree. This mutation was not found on 150 control Caucasian chromosomes.

The G285S mutation affects the first glycine in the GYG signature sequence of potassium channel pores. This glycine is highly conserved across different classes of potassium channels in all species. The crystal structure of the *Streptomyces lividans* potassium channel reveals that these three amino acids line the narrowest part of the ion-conductive pore. Mutations in these amino-acids disrupt the selectivity filter and in most cases lead to a loss of channel function. Interestingly, an identical change in amino acids at the equivalent position was found in the *KCNQ1* gene of a patient with the dominant long QT syndrome. It disrupted channel activity and exerted a dominant negative effect on co-expressed WT KCNQ1 channels. These mutations also have dominant negative effects when inserted into KCNQ2 and *KCNQ3.* This is strong evidence that the progressive hearing loss in this family is due to the KCNQ4/G285S mutation.

### Example 4

### Functional expression of KCNQ4 potassium channel subunits

KCNQ4 was expressed in *Xenopus* oocytes and its activity was investigated by two-electrode voltage clamping.

After linearization of the *KCNQ4*-containing PTLN vector with Hpal, capped cRNA was transcribed *in vitro* using the mMessage mMachine cRNA synthesis kit (Ambion). Usually 5 - 15 ng of cRNA were injected into *Xenopus* oocytes previously isolated by manual defolliculation and short collagenase treatment. In co-expression experiments cRNAs were injected at a 1:1 ratio. Oocytes were kept at 17°C in modified Barth's solution (90 mM NaCl, 1 mM KCl, 0.41 mM CaCl₂, 0.33 mM Ca(NO₃)₂, 0.82 mM MgSO₄, 10 mM HEPES, 100 U penicillin-100 µg streptomycin/ml, pH 7.6).

Standard two-electrode voltage-clamp measurements were performed at room temperature 2-4 days after injection using a Turbotec 05 amplifier (npi instruments, Tamm, Germany) and pClamp 5.5 software (Axon Instruments). Currents were usually recorded in ND98 solution (see Table 2). Solutions for Na⁺ / K⁺ replacement experiments were prepared from an appropriate mixture of solution KD100 (100 mM KCl) and ND100 (100 mM NaCl) to yield the stated concentrations of Na⁺ and K⁺. Linopirdine (RBI, Natick, MA) was prepared as a 100 mM stock solution in DMSO and added to a final concentration of 200 µM to ND98.

**Table 2**

| **Solution contents (concentrations in mM)** | | | | |
|---|---|---|---|---|
| ND98 | ND 100 | KD100 | Rb100 | Cs100 |
| 98 NaCl | 100 NaCl | 100 KCl | 100 RbCl | 100 CsCl |
| 2 KCl | | | | |
| 0.2 CaCl₂ | 0.2 CaCl₂ | 0.2 CaCl₂ | 0.2 CaCl₂ | 0.2 CaCl₂ |
| 2.8 MgCl₂ | 2.8 MgCl₂ | 2.8 MgCl₂ | 2.8 MgCl₂ | 2.8 MgCl₂ |
| 5 mM HEPES, pH 7.4 | | | | |

Reversal potentials were determined from tail currents after a 2 s depolarising pulse to +60 mV and corrected for liquid junction potentials that were determined experimentally. The permeability ratios were calculated according to P_{K} / Px = exp (-F·Vᵣₑᵥ / R·T).

To determine the voltage dependence of apparent open probability, oocytes were clamped for 4 s to values between -80 mV to +50 mV in 10 mV steps, followed by a constant -30 mV test pulse. Tail currents extrapolated to t=0 were obtained from a mono-exponential fit, normalised to the value at 0 mV and used for the analysis of apparent pₒₚₑₙ. Data analysis used PClamp6 and Microcal Origin 5.0.

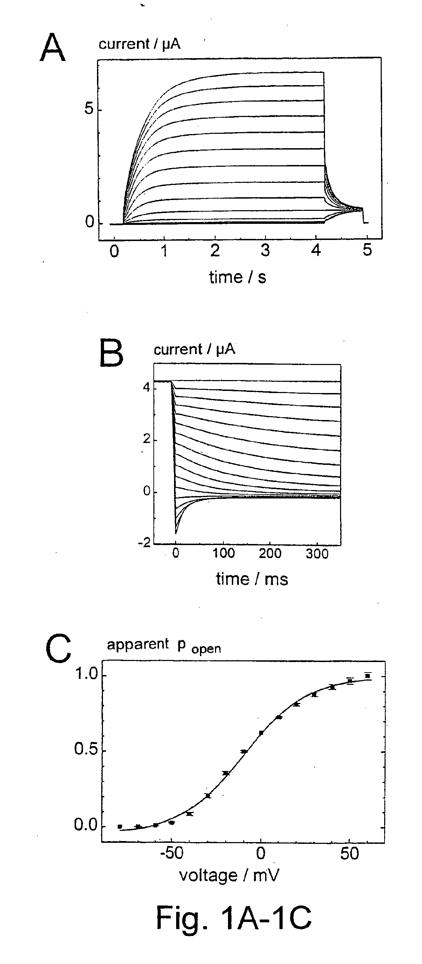

Similar to KCNQ1, KCNQ2 and KCNQ3, also KCNQ4 yielded currents that activated upon depolarisation (Fig. 1A). Compared to those other KCNQ channels, however, current activation was slower and occurred with a time constant in the order of 600 ms at + 40mV (KCNQ2/KCNQ3 channels have a corresponding time constant of ≈300 ms). This time constant was very sensitive to changes in temperature. Deactivation of currents at physiological resting potentials (≈-70mV) was considerably faster (Fig. 1 B). Similar to KCNQ2, macroscopic currents often showed some inward rectification at positive potentials. When oocytes were depolarised to +60 mV for 10 sec or more, an apparent slow inactivation of currents was observed that resembled the one described for KCNQ3. Currents began to activate at about -40 mV, with half-maximal activation at -10 mV (Fig. 2C). Ion substitution experiments showed that the channel is highly selective for potassium (Fig. 1 D). It has a K⁺≈Rb⁺>Cs⁺>Na⁺ permeability sequence. KCNQ4 currents were inhibited by more than 80% by 5 mM Ba⁺⁺.

We next examined the effect of the G285S mutation found in the affected family (Figs. 1 E and 1 F). The mutant channel did not yield any detectable currents in the *Xenopus* oocyte expression system. KCNQ4_{G285S} was then injected at a 1:1 ratio with WT KCNQ4 to mimic the situation in a heterozygous DFNA2 patient. This reduced currents by about 90%, indicating a strong dominant negative effect of the mutant. The degree of current reduction is compatible with the notion that the incorporation of one mutant subunit suffices to abolish the function of the tetrameric channel complex. The channels present in co-injected oocytes still showed a strong preference of potassium over sodium or calcium. This implies that the deafness is due to a quantitative loss of KCNQ4 potassium currents rather than to an influx of sodium or calcium.

KCNQ1 assembles with minK (IsK) to form channels that yield larger currents and activate much slower. We therefore tested by co-expression whether minK affects KCNQ4 as well. At concentrations (1 ng minK cRNA per oocyte) leading to drastic changes in KCNQ1 currents in parallel experiments, there was no significant change in KCNQ4 currents.

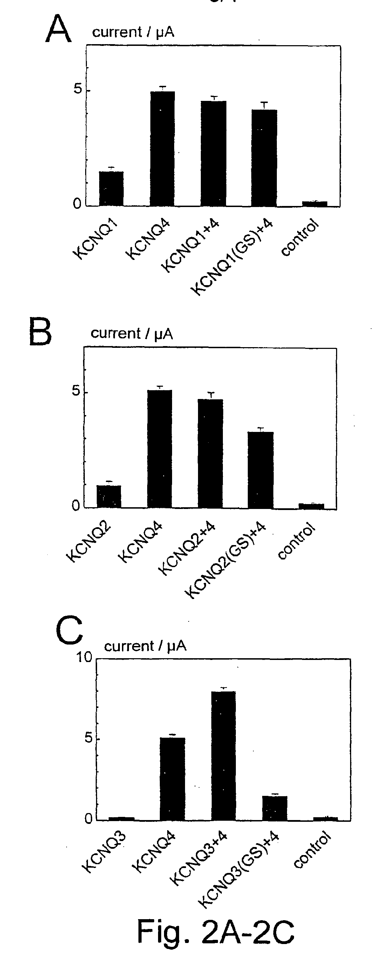

Different KCNQ subunits can form heteromeric channels. Co-expression of KCNQ2 with KCNQ3, but not with KCNQ1, gave currents that were about tenfold larger than those from homomeric channels. Since also KCNQ1 and KCNQ3 (and to a lesser degree also KCNQ2) are expressed in the cochlea, we investigated whether these proteins interact functionally. Oocytes co-injected (at the same total cRNA concentration) with KCNQ1 and KCNQ4 cRNAs yielded currents that seemed not different from a linear superposition of currents from the respective homomeric channels (Fig. 2A), and the same was true for oocytes co-expressing KCNQ2 and KCNQ4 (Fig. 2B). In addition, a dominant negative KCNQ1 mutant did not suppress KCNQ4 currents (Fig. 2A), and the same was true for the equivalent KCNQ2 mutant (Fig. 2B).

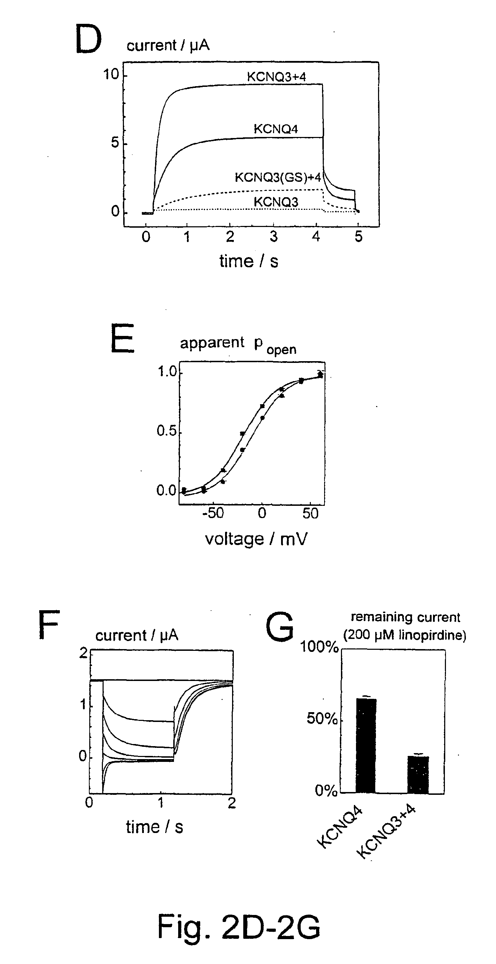

By contrast, co-expression of KCNQ3 with KCNQ4 yielded currents that were significantly larger than could be explained by a superposition of currents from the respective homomeric channels (Figs. 2C and 2D). Further, KCNQ4 currents were markedly suppressed by co-expressing a dominant negative KCNQ3 mutant (Fig. 2C). Importantly, currents from co-injected oocytes activated faster than KCNQ4 currents (Fig. 2D), and there was a =10 mV shift of the open probability towards negative voltages (Fig. 2E). Compared to KCNQ2/KCNQ3 channels, which may underlie the M-current, KCNQ3/KCNQ4 heteromers open at slightly more positive voltages. To compare KCNQ3/KCNQ4 channels to M-channels, we used the typical voltage-protocol employed for these channels and found currents superficially resembling M-currents (Fig. 2F). Linopirdine, a potent and rather specific inhibitor for M-currents, nearly completely inhibits KCNQ2/KCNQ3 channels at a concentration of 200 µM. This concentration of Linopirdine inhibited KCNQ4 by about 30%, while a significantly larger inhibition (≈75%) was observed with KCNQ3/KCNQ4 co-expression (Fig. 2G).

### SEQUENCE LISTING

<160> 32
<170> PatentIn Ver. 2.1
<210> 1
   <211> 2335
   <212> DNA
   <213> Homo sapiens
<220>
   <221> gene
   <222> (1)..(2335)
<220>
   <221> CDS
   <222> (83)..(2170)
   <223> KCNQ4
<400> 1
   agccatgcgt ctctgagcgc cccgagcgcg cccccgcccc ggaccgtgcc cgggccccgg 60
   cgcccccagc ccggcgccgc cc atg gcc gag gcc ccc ccg cgc cgc ctc ggc  112
   ctg ggt ccc ccg ccc ggg gac gcc ccc cgc gcg gag cta gtg gcg ctc   160
   acg gcc gtg cag agc gaa cag ggc gag gcg ggc ggg ggc ggc tcc ccg   208
   cgc cgc ctc ggc ctc ctg ggc agc ccc ctg ccg ccg ggc gcg ccc ctc   256
   cct ggg ccg ggc tcc ggc tcg ggc tcc gcc tgc ggc cag cgc tcc tcg    304
   gcc gcg cac aag cgc tac cgc cgc ctg cag aac tgg gtc tac aac gtg    352
   ctg gag cgg ccc cgc ggc tgg gcc ttc gtc tac cac gtc ttc ata ttt    400
   ttg ctg gtc ttc agc tgc ctg gtg ctg tct gtg ctg tcc act atc cag    448
   gag cac cag gaa ctt gcc aac gag tgt ctc ctc atc ttg gaa ttc gtg    496
   atg atc gtg gtt ttc ggc ttg gag tac atc gtc cgg gtc tgg tcc gcc    544
   gga tgc tgc tgc cgc tac cga gga tgg cag ggt cgc ttc cgc ttt gcc    592
   aga aag ccc ttc tgt gtc atc gac ttc atc gtg ttc gtg gcc tcg gtg    640
   gcc gtc atc gcc gcg ggt acc cag ggc aac atc ttc gcc acg tcc gcg    688
   ctg cgc agc atg cgc ttc ctg cag atc ctg cgc atg gtg cgc atg gac    736
   cgc cgc ggc ggc acc tgg aag ctg ctg ggc tca gtg gtc tac gcg cat 784
   agc aag gag ctg atc acc gcc tgg tac atc ggg ttc ctg gtg ctc atc 832
   ttc gcc tcc ttc ctg gtc tac ctg gcc gag aag gac gcc aac tcc gac 880
   ttc tcc tcc tac gcc gac tcg ctc tgg tgg ggg acg att aca ttg aca 928
   acc atc ggc tat ggt gac aag aca ccg cac aca tgg ctg ggc agg gtc 976
   ctg gct gct ggc ttc gcc tta ctg ggc atc tct ttc ttt gcc ctg cct 1024
   gcc ggc atc cta ggc tcc ggc ttt gcc ctg aag gtc cag gag cag cac 1072
   cgg cag aag cac ttc gag aag cgg agg atg ccg gca gcc aac ctc atc 1120
   cag gct gcc tgg cgc ctg tac tcc acc gat atg agc cgg gcc tac ctg 1168
   aca gcc acc tgg tac tac tat gac agt atc ctc cca tcc ttc aga gag 1216
   ctg gcc ctc ttg ttt gag cac gtg caa cgg gcc cgc aat ggg ggc cta 1264
   cgg ccc ctg gag gtg cgg cgg gcg ccg gta ccc gac gga gca ccc tcc 1312
   cgt tac ccg ccc gtt gcc acc tgc cac cgg ccg ggc agc acc tcc ttc 1360
   tgc cct ggg gaa agc agc cgg atg ggc atc aaa gac cgc atc cgc atg 1408
   ggc agc tcc cag cgg cgg acg ggt cct tcc aag cag cag ctg gca cct 1456
   cca aca atg ccc acc tcc cca agc agc gag cag gtg ggt gag gcc acc 1504
   agc ccc acc aag gtg caa aag agc tgg agc ttc aat gac cgc acc cgc 1552
   ttc cgg gca tct ctg aga ctc aaa ccc cgc acc tct gct gag gat gcc 1600
   ccc tca gag gaa gta gca gag gag aag agc tac cag tgt gag ctc acg 1648
   gtg gac gac atc atg cct gct gtg aag aca gtc atc cgc tcc atc agg 1696
   att ctc aag ttc ctg gtg gcc aaa agg aaa ttc aag gag aca ctg cga 1744
   ccg tac gac gtg aag gac gtc att gag cag tac tca gca ggc cac ctg 1792
   gac atg ctg ggc cgg atc aag agc ctg caa act cgg gtg gac caa att 1840
   gtg ggt cgg ggg ccc ggg gac agg aag gcc cgg gag aag ggc gac aag 1888
   ggg ccc tcc gac gcg gag gtg gtg gat gaa atc agc atg atg gga cgc 1936
   gtg gtc aag gtg gag aag cag gtg cag tcc atc gag cac aag ctg gac 1984
   ctg ctg ttg ggc ttc tat tcg cgc tgc ctg cgc tct ggc acc tcg gcc 2032
   agc ctg ggc gcc gtg caa gtg ccg ctg ttc gac ccc gac atc acc tcc 2080
   gac tac cac agc cct gtg gac cac gag gac atc tcc gtc tcc gca cag 2128
   acg ctc agc atc tcc cgc tcg gtc agc acc aac atg gac tga 2170
   gggacttctc agaggcaggg cagcacacgg ccagccccgc ggcctggcgc tccgactgcc 2230
   ctctgaggcc tccggactcc tctcgtactt gaactcactc cctcacgggg agagagacca 2290
   cacgcagtat tgagctgcct gagtgggcgt ggtacctgct gtggg 2335
<210> 2
   <211> 695
   <212> PRT
   <213> Homo sapiens
<400> 2
<210> 3
   <211> 24
   <212> DNA
   <213> Artificial Sequence
<220>
   <223> Description of Artificial Sequence: PCR primer
<400> 3
   catgcgtctc tgagcgcccc gagc 24
<210> 4
   <211> 24
   <212> DNA
   <213> Artificial Sequence
<220>
   <223> Description of Artificial Sequence: PCR primer
<400> 4
   aggccaggct tgcgcgggga aacg 24
<210> 5
   <211> 23
   <212> DNA
   <213> Artificial Sequence
<220>
   <223> Description of Artificial Sequence: PCR primer
<400> 5
   cagcacagag ctgtaactcc agg 23
<210> 6
   <211> 21
   <212> DNA
   <213> Artificial Sequence
<220>
   <223> Description of Artificial Sequence: PCR primer
<400> 6
   aagctgctct ctgagccatg g 21
<210> 7
   <211> 20
   <212> DNA
   <213> Artificial Sequence
<220>
   <223> Description of Artificial Sequence: PCR primer
<400> 7
   gctgggtccg cgctgtgacc 20
<210> 8
   <211> 20
   <212> DNA
   <213> Artificial Sequence
<220>
   <223> Description of Artificial Sequence: PCR primer
<400> 8
   ggtctccagg gtcagagtcg 20
<210> 9
   <211> 21
   <212> DNA
   <213> Artificial Sequence
<220>
   <223> Description of Artificial Sequence: PCR primer
<400> 9
   tccgggtccg tgcgcggggt a 21
<210> 10
   <211> 21
   <212> DNA
   <213> Artificial Sequence
<220>
   <223> Description of Artificial Sequence: PCR primer
<400> 10
   gagacagccc ctctgacctc g 21
<210> 11
   <211> 21
   <212> DNA
   <213> Artificial Sequence
<220>
   <223> Description of Artificial Sequence: PCR primer
<400> 11
   atccctttcc cgtgtggaag c 21
<210> 12
   <211> 21
   <212> DNA
   <213> Artificial Sequence
<220>
   <223> Description of Artificial Sequence: PCR primer
<400> 12
   agtcacgatg ggcagacctc g 21
<210> 13
   <211> 21
   <212> DNA
   <213> Artificial Sequence
<220>
   <223> Description of Artificial Sequence: PCR primer
<400> 13
   cctcatgatc aggctcctac c 21
<210> 14
   <211> 19
   <212> DNA
   <213> Artificial Sequence
<220>
   <223> Description of Artificial Sequence: PCR primer
<400> 14
   atgtgtgaca ggggtgagc 19
<210> 15
   <211> 20
   <212> DNA
   <213> Artificial Sequence
<220>
   <223> Description of Artificial Sequence: PCR primer
<400> 15
   aaggatgggg acacccttgc 20
<210> 16
   <211> 18
   <212> DNA
   <213> Artificial Sequence
<220>
   <223> Description of Artificial Sequence: PCR primer
<400> 16
   acacagggtt gacacacc 18
<210> 17
   <211> 21
   <212> DNA
   <213> Artificial Sequence
<220>
   <223> Description of Artificial Sequence: PCR primer
<400> 17
   gctctgggta acccacaact g 21
<210> 18
   <211> 21
   <212> DNA
   <213> Artificial Sequence
<220>
   <223> Description of Artificial Sequence: PCR primer
<400> 18
   gctcccctgg gagccatcac c 21
<210> 19
   <211> 20
   <212> DNA
   <213> Artificial Sequence
<220>
   <223> Description of Artificial Sequence: PCR primer
<400> 19
   tgagctcagg agctctgtgc 20
<210> 20
   <211> 21
   <212> DNA
   <213> Artificial Sequence
<220>
   <223> Description of Artificial Sequence: PCR primer
<400> 20
   acccacgaag tggctgaagg c 21
<210> 21
   <211> 20
   <212> DNA
   <213> Artificial Sequence
<220>
   <223> Description of Artificial Sequence: PCR primer
<400> 21
   gtcctaagtc agctttgtcc 20
<210> 22
   <211> 20
   <212> DNA
   <213> Artificial Sequence
<220>
   <223> Description of Artificial Sequence: PCR primer
<400> 22
   cctcagccgg ccctcgatcg 20
<210> 23
   <211> 20
   <212> DNA
   <213> Artificial Sequence
<220>
   <223> Description of Artificial Sequence: PCR primer
<400> 23
   cactctactg gtggtttggc 20
<210> 24
   <211> 20
   <212> DNA
   <213> Artificial Sequence
<220>
   <223> Description of Artificial Sequence: PCR primer
<400> 24
   ctcctgacct caagtgatcc 20
<210> 25
   <211> 21
   <212> DNA
   <213> Artificial Sequence
<220>
   <223> Description of Artificial Sequence: PCR primer
<400> 25
   gatagcaaag agatggagag g 21
<210> 26
   <211> 21
   <212> DNA
   <213> Artificial Sequence
<220>
   <223> Description of Artificial Sequence: PCR primer
<400> 26
   aactcagctg cagcagtgag c 21
<210> 27
   <211> 21
   <212> DNA
   <213> Artificial Sequence
<220>
   <223> Description of Artificial Sequence: PCR primer
<400> 27
   gtgccttctc cttcatcagg c 21
<210> 28
   <211> 20
   <212> DNA
   <213> Artificial Sequence
<220>
   <223> Description of Artificial Sequence: PCR primer
<400> 28
   aacgcatcct ccccatgtca 20
<210> 29
   <211> 20
   <212> DNA
   <213> Artificial Sequence
<220>
   <223> Description of Artificial Sequence: PCR primer
<400> 29
   tttgtgcttc ccagataagc 20
<210> 30
   <211> 24
   <212> DNA
   <213> Artificial Sequence
<220>
   <223> Description of Artificial Sequence: PCR primer
<400> 30
   cgtgagggag tgagttcaag tacg 24
<210> 31
   <211> 24
   <212> DNA
   <213> Artificial Sequence
<220>
   <223> Description of Artificial Sequence: PCR primer
<400> 31
   agtacctgat ggagcgccct ctcg 24
<210> 32
   <211> 24
   <212> DNA
   <213> Artificial Sequence
<220>
   <223> Description of Artificial Sequence: PCR primer
<400> 32
   tcatccaccg taagctcaca ctgg 24

## Claims

1. An isolated polynucleotide having a nucleic acid sequence which is capable of hybridising under high stringency conditions with the polynucleotide sequence presented as SEQ ID NO: 1, or its complementary strand, wherein said hybridization occurs in a solution of 5 x SSC, 5 x Denhardt's solution, 0.5 % SDS and 100 µg/ml of denatured sonicated salmon sperm DNA for 12 hours at approximately 45°C, followed by washing twice for 30 minutes in 2 x SSC, 0.5 % SDS at a temperature of at least 65°C, encoding a potassium channel KCNQ4 subunit.

2. The isolated polynucleotide according to claim 1, wherein said hybridization is followed by washing twice for 30 minutes in 2 x SSC, 0.5 % SDS at a temperature of at least at least 70°C.

3. The isolated polynucleotide according to claim 1, wherein said hybridization is followed by washing twice for 30 minutes in 2 x SSC, 0.5 % SDS at a temperature of at least at least 75°C.

4. The isolated polynucleotide according to any one of claims 1-3, being at least 90% homologous to the polynucleotide sequence presented as SEQ ID NO: 1.

5. The isolated polynucleotide according to claim 4, being at least 95% homologous to the polynucleotide sequence presented as SEQ ID NO: 1.

6. The isolated polynucleotide according to any one of claims 1-5 being a cloned polynucleotide.

7. The isolated polynucleotide according to claim 6, in which the polynucleotide is cloned from, or produced on the basis of a cDNA library.

8. The isolated polynucleotide according to any one of claims 1-7, comprising the polynucleotide sequence presented as SEQ ID NO: 1.

9. The isolated polynucleotide according to any one of claims 1-7, comprising the polynucleotide sequence presented as SEQ ID NO: 1, including the mutation G935A.

10. The isolated polynucleotide according to claim 1, encoding the KCNQ4 potassium channel subunit comprising the amino acid sequence represented by SEQ ID NO: 2.

11. The isolated polynucleotide according to claim 1, encoding a KCNQ4 variant, which variant has an amino acid sequence that has been changed by deletion of an amino acid residue, by insertion of an additional amino acid residue, or by substitution of an amino acid residue at one or more positions located in the conserved regions, as defined by Table 1.

12. The isolated polynucleotide according to claim 11, encoding the variant G285S of SEQ ID NO: 2.

13. A recombinantly produced polypeptide encoded by the polynucleotide according to any one of claims 1-12.

14. The polypeptide according to claim 13, being a KCNQ4 potassium channel subunit comprising the amino acid sequence presented as SEQ ID No. 2.

15. The polypeptide according to claim 13, being a KCNQ4 variant, which variant has an amino acid sequence that has been changed by deletion of an amino acid residue, by insertion of an additional amino acid residue, or by substitution of an amino acid residue at one or more positions located in the conserved regions, as defined by Table 1.

16. The polypeptide according to claim 15, being the variant G285S of SEQ ID NO: 2.

17. A cell genetically manipulated by the incorporation of a heterologous polynucleotide according to any one of claims 1-12.

18. The cell according to claim 17, genetically manipulated by the incorporation of a KCNQ4 channel subunit comprising the amino acid sequence presented as SEQ ID NO: 2.

19. The cell according to claim 18, genetically manipulated by the incorporation of a KCNQ4 variant, which variant has an amino acid sequence that has been changed by deletion of an amino acid residue, by insertion of an additional amino acid residue, or by substitution of an amino acid residue at one or more positions located in the conserved regions, as defined by Table 1.

20. The cell according to claim 19, genetically manipulated by the incorporation of the variant G285S of SEQ ID NO: 2.

21. The cell according to any one of claims 17-20, genetically manipulated to co-express one or more KCNQ channel subunits.

22. The cell according to claim 21, genetically manipulated to co-express the KCNQ4 polypeptide according to any one of claims 13-16 and KCNQ1 channel subunits; the KCNQ4 polypeptide according to any one of claims 13-16 and KCNQ2 channel subunits; the KCNQ4 polypeptide according to any one of claims 13-16 and KCNQ3 channel subunits; the KCNQ4 polypeptide according to any one of claims 13-16 and KCNQ1 and KCNQ2 channel subunits; the KCNQ4 polypeptide according to any one of claims 13-16 and KCNQ1 and KCNQ3 channel subunits; the KCNQ4 polypeptide according to any one of claims 13-16 and KCNQ2 and KCNQ3 channel subunits; or the KCNQ4 polypeptide according to any one of claims 13-16 and KCNQ1 and KCNQ2 and KCNQ3 channel subunits.

23. The cell according to claim 22, genetically manipulated to co-express KCNQ3 and the KCNQ4 channel subunits according to any one of claims 13-16.

24. The cell according to any one of claims 17-23, being a eukaryotic cell, in particular a mammalian cell, an oocyte, or a yeast cell.

25. The cell according to claim 24, being a human embryonic kidney (HEK) cell, a HEK 293 cell, a BHK21 cell, a Chinese hamster ovary (CHO) cell, a *Xenopus laevis* oocyte (XLO) cell, or any other cell line able to express KCNQ potassium channels.

26. A method of screening a chemical compound for its capability of binding to a potassium channel comprising at least one KCNQ4 channel subunit, which method comprises the steps of
(i) subjecting the KCNQ4 channel subunit containing cell according to any one of claims 17-25 to the action of an agent capable of binding to the KCNQ4 polypeptide, thereby forming a complex with the KCNQ4 channel subunit containing cell, in which said agent capable of binding to the KCNQ4 polypeptide is radioactively labelled 1,3-dihydro-1-phenyl-3,3-bis(4-pyridylmethyl)-2H-indol-2-one (Linopirdine); or radioactively labelled 10,10-bis(4-pyridinyl-methyl)-9(10H)-antracenone.
(ii) subjecting the complex of step (i) to the action of the chemical compound to be tested; and
(iii) detecting the displacement of said agent capable of binding to the KCNQ4 polypeptide from the complex with the KCNQ4 channel subunit containing cell.

27. The method of claim 26, in which said agent has been radioactively labelled with ³H.

28. The method of either one of claims 26-27, wherein the displacement of said agent capable of binding to the KCNQ4 polypeptide from the complex with the KCNQ4 channel subunit containing cell is detected by measuring the amount of radioactivity by conventional liquid scintillation counting.

29. A method of screening a chemical compound for activity on a potassium channel comprising at least one KCNQ4 channel subunit, which method comprises the steps of
(i) subjecting a KCNQ4 channel subunit containing cell according to any one of claims 17-25 to the action of the chemical compound; and
(ii) monitoring the membrane potential, the current, the potassium flux, or the secondary calcium influx of the KCNQ4 channel subunit containing cell.

30. The method of claim 29, wherein monitoring of the membrane potential of the KCNQ4 channel subunit containing cell is performed by patch clamp techniques.

31. The method of claim 30, wherein monitoring of the membrane potential of the KCNQ4 channel subunit containing cell is performed using fluorescence methods.

32. Use of a polynucleotide sequence according to claim 9, for the screening of genetic materials for individuals having this mutation.

33. A transgenic rodent, a transgenic pig, a transgenic cattle, a transgenic sheep, or a transgenic goat comprising a knock-out mutation of the endogenous *KCNQ4* polynucleotide according to any one of claims 1-12, a mutated *KCNQ4* polynucleotide according to any one of claims 9, 11 or 12, or genetically manipulated in order to over-express the *KCNQ4* polynucleotide according to any one of claims 1-12, or to over-express mutated *KCNQ4* polynucleotide according to any one of claims 9, 11 or 12.

34. The transgenic animal according to claim 33, being a knock-out animal in which the KCNQ4 polynucleotide is totally deleted in a homozygous state.

35. The transgenic animal according to claim 33, comprising a mutated *KCNQ4* polynucleotide according to any one of claims 9, 11 or 12.

36. The transgenic animal according to claim 35, comprising the mutated *KCNQ4* polynucleotide according to claim 9.

37. The transgenic animal according to any one of claims 33-36 being a transgenic hamster, a guinea pig, a rabbit, or a rat.

38. Use of the transgenic animal according to any one of claims 33-37 for the *in vivo* screening of therapeutic compounds.

39. The use according to claim 38, for the screening of drugs affecting diseases or conditions associated with hearing loss or tinnitus.

## Patentansprüche

1. Isoliertes, für eine KCNQ4-Kaliumkanaluntereinheit codierendes Polynukleotid mit einer Nukleinsäuresequenz, die unter hoch stringenten Bedingungen mit der als SEQ ID NO. 1 dargestellten Polynukleotidsequenz oder ihrem Komplementärstrang hybridisieren kann, wobei besagte Hybridisierung in einer Lösung aus 5 x SSC, 5 x Denhardts Lösung, 0,5 % SDS und 100 µg/ml denaturierter, beschallter Lachsspermien-DNA über 12 Stunden bei etwa 45°C erfolgt, gefolgt von zweimaligem Waschen über 30 Minuten in 2 x SSC, 0,5 % SDS bei einer Temperatur von mindestens 65°C.

2. Isoliertes Polynukleotid nach Anspruch 1, wobei auf besagte Hybridisierung zweimaliges Waschen über 30 Minuten in 2 x SSC, 0,5 % SDS bei einer Temperatur von mindestens 70°C folgt.

3. Isoliertes Polynukleotid nach Anspruch 1, wobei auf besagte Hybridisierung zweimaliges Waschen über 30 Minuten in 2 x SSC, 0,5 % SDS bei einer Temperatur von mindestens 75°C folgt.

4. Isoliertes Polynukleotid nach einem der Ansprüche 1-3, das zu mindestens 90 % mit der als SEQ ID NO. 1 dargestellten Polynukleotidsequenz homolog ist.

5. Isoliertes Polynukleotid nach Anspruch 4, das zu mindestens 95 % mit der als SEQ ID NO. 1 dargestellten Polynukleotidsequenz homolog ist.

6. Isoliertes Polynukleotid nach einem der Ansprüche 1-5, welches ein kloniertes Polynukleotid ist.

7. Isoliertes Polynukleotid nach Anspruch 6, wobei das Polynukleotid aus einer cDNA-Bibliothek kloniert oder auf Basis einer cDNA-Bibliothek hergestellt wird.

8. Isoliertes Polynukleotid nach einem der Ansprüche 1-7, welches die als SEQ ID NO. 1 dargestellte Polynukleotidsequenz umfasst.

9. Isoliertes Polynukleotid nach einem der Ansprüche 1-7, welches die als SEQ ID NO. 1 dargestellte Polynukleotidsequenz einschließlich der Mutation G935A umfasst.

10. Isoliertes Polynukleotid nach Anspruch 1, das für die KCNQ4-Kaliumkanaluntereinheit, welche die als SEQ ID NO. 2 dargestellte Aminosäuresequenz umfasst, codiert.

11. Isoliertes Polynukleotid nach Anspruch 1, das für eine KCNQ4-Variante codiert, wobei diese Variante eine Aminosäuresequenz hat, die durch Deletion eines Aminosäurerestes, durch Insertion eines zusätzlichen Aminosäurerestes oder durch Substitution eines Aminosäurerestes an einer oder mehreren in den konservierten Regionen gelegenen Positionen wie in Tabelle 1 definiert geändert wurde.

12. Isoliertes Polynukleotid nach Anspruch 11, das für die Variante G285S von SEQ ID NO. 2 codiert.

13. Rekombinant hergestelltes Polypeptid, für das ein Polynukleotid nach einem der Ansprüche 1-12 codiert.

14. Polypeptid nach Anspruch 13, das eine KCNQ4-Kaliumkanaluntereinheit, welche die als SEQ ID NO. 2 dargestellte Aminosäuresequenz umfasst, ist.

15. Polypeptid nach Anspruch 13, das eine KCNQ4-Variante ist, wobei diese Variante eine Aminosäuresequenz hat, die durch Deletion eines Aminosäurerestes, durch Insertion eines zusätzlichen Aminosäurerestes oder durch Substitution eines Aminosäurerestes an einer oder mehreren in den konservierten Regionen gelegenen Positionen wie in Tabelle 1 definiert geändert wurde.

16. Polypeptid nach Anspruch 15, das die Variante G285S von SEQ ID NO. 2 ist.

17. Zelle, die durch das Einbringen eines heterologen Polynukleotids nach einem der Ansprüche 1-12 genetisch manipuliert ist.

18. Zelle nach Anspruch 17, die durch das Einbringen einer KCNQ4-Kanaluntereinheit, welche die als SEQ ID NO. 2 dargestellte Aminosäuresequenz umfasst, genetisch manipuliert ist.

19. Zelle nach Anspruch 18, die durch das Einbringen einer KCNQ4-Variante genetisch manipuliert ist, wobei diese Variante eine Aminosäuresequenz hat, die durch Deletion eines Aminosäurerestes, durch Insertion eines zusätzlichen Aminosäurerestes oder durch Substitution eines Aminosäurerestes an einer oder mehreren in den konservierten Regionen gelegenen Positionen wie in Tabelle 1 definiert geändert wurde.

20. Zelle nach Anspruch 19, die durch das Einbringen der Variante G285S von SEQ ID NO. 2 genetisch manipuliert ist.

21. Zelle nach einem der Ansprüche 17-20, die genetisch manipuliert ist, um eine oder mehrere KCNQ-Kanaluntereinheiten zusammen zu exprimieren.

22. Zelle nach Anspruch 21, die genetisch manipuliert ist, um das Polypeptid KCNQ4 nach einem der Ansprüche 13-16 und KCNQ1-Kanaluntereinheiten, das Polypeptid KCNQ4 nach einem der Ansprüche 13-16 und KCNQ2-Kanaluntereinheiten, das Polypeptid KCNQ4 nach einem der Ansprüche 13-16 und KCNQ3-Kanaluntereinheiten, das Polypeptid KCNQ4 nach einem der Ansprüche 13-16 und KCNQ1- und KCNQ2-Kanaluntereinheiten, das Polypeptid KCNQ4 nach einem der Ansprüche 13-16 und KCNQ1- und KCNQ3-Kanaluntereinheiten, das Polypeptid KCNQ4 nach einem der Ansprüche 13-16 und KCNQ2- und KCNQ3-Kanaluntereinheiten oder das Polypeptid KCNQ4 nach einem der Ansprüche 13-16 und KCNQ1- und KCNQ2- und KCNQ3-Kanaluntereinheiten zusammen zu exprimieren.

23. Zelle nach Anspruch 22, die genetisch manipuliert ist, um KCNQ3 und die KCNQ4-Kanaluntereinheiten nach einem der Ansprüche 13-16 zusammen zu exprimieren.

24. Zelle nach einem der Ansprüche 17-23, die eine eukaryotische Zelle, im Besonderen eine Säugetierzelle, eine Eizelle oder eine Hefezelle ist.

25. Zelle nach Anspruch 24, die eine Nierenzelle vom menschlichen Embryo (HEK), eine HEK 293 Zelle, eine BHK21 Zelle, eine Ovarzelle vom chinesischen Hamster (CHO), eine Eizelle von *Xenopus laevis* (XLO) oder jede andere Zelllinie, die KCNQ-Kaliumkanäle exprimieren kann, ist.

26. Verfahren zum Überprüfen einer chemischen Verbindung auf ihre Fähigkeit, an einen Kaliumkanal, der mindestens eine KCNQ4-Kanaluntereinheit umfasst, zu binden, wobei dieses Verfahren die folgenden Schritte umfasst:
(i) Aussetzen der eine KCNQ4-Kanaluntereinheit enthaltenden Zelle nach einem der Ansprüche 17-25 der Wirkung eines Wirkstoffes, der an das Polypeptid KCNQ4 binden kann, und dabei Bilden eines Komplexes mit der eine KCNQ4-Kanaluntereinheit enthaltenden Zelle, wobei besagter Wirkstoff, der an das Polypeptid KCNQ4 binden kann,
radioaktiv markiertes 1,3-Dihydro-1-phenyl-3,3-bis(4-pyridylmethyl)-2H-indol-2-on (Linopirdin) oder
radioaktiv markiertes 10,10-Bis(4-pyridinyl-methyl)-9(10H)-anthracenon ist,
(ii) Aussetzen des Komplexes aus Schritt (i) der Wirkung der zu prüfenden chemischen Verbindung und
(iii) Nachweisen der Verdrängung des besagten Wirkstoffes, der an das Polypeptid KCNQ4 binden kann, vom Komplex mit der eine KCNQ4-Kanaluntereinheit enthaltenden Zelle.

27. Verfahren nach Anspruch 26, worin besagter Wirkstoff mit ³H radioaktiv markiert wurde.

28. Verfahren nach einem der Ansprüche 26-27, worin die Verdrängung des besagten Wirkstoffs, der an das Polypeptid KCNQ4 binden kann, vom Komplex mit der eine KCNQ4-Kanaluntereinheit enthaltenden Zelle durch Messen der Menge der Radioaktivität mittels herkömmlicher Flüssigkeitsszintillationszählung nachgewiesen wird.

29. Verfahren zum Überprüfen einer chemischen Verbindung auf Aktivität auf einen Kaliumkanal, der mindestens eine KCNQ4-Untereinheit umfasst, wobei dieses Verfahren die folgenden Schritte umfasst:
(i) Aussetzen der eine KCNQ4-Kanaluntereinheit enthaltenden Zelle nach einem der Ansprüche 17-25 der Wirkung der chemischen Verbindung und
(ii) Überwachen des Membranpotentials, des Stroms, des Kaliumstroms oder des sekundären Calciumeinstroms der eine KCNQ4-Kanaluntereinheit enthaltenden Zelle.

30. Verfahren nach Anspruch 29, worin das Überwachen des Membranpotentials der eine KCNQ4-Kanaluntereinheit enthaltenden Zelle mittels Membranfleckklemmentechniken erfolgt.

31. Verfahren nach Anspruch 30, worin das Überwachen des Membranpotentials der eine KCNQ4-Kanaluntereinheit enthaltenden Zelle unter Verwendung von Fluoreszenzverfahren erfolgt.

32. Verwendung einer Polynukleotidsequenz nach Anspruch 9 zum Überprüfen von genetischen Materialien auf Individuen, die diese Mutation haben.

33. Transgener Nager, transgenes Schwein, transgenes Rind, transgenes Schaf oder transgene Ziege, welche(r/s) eine Knock-out-Mutation des endogenen Polynukleotids KCNQ4 nach einem der Ansprüche 1-12 oder ein mutiertes Polynukleotid *KCNQ4* nach einem der Ansprüche 9, 11 oder 12 umfasst oder genetisch manipuliert ist, um das Polynukleotid *KCNQ4* nach einem der Ansprüche 1-12 übermäßig zu exprimieren oder um das mutierte Polynukleotid *KCNQ4* nach einem der Ansprüche 9, 11 oder 12 übermäßig zu exprimieren.

34. Transgenes Tier nach Anspruch 33, welches ein Knock-out-Tier, in dem das Polynukleotid KCNQ4 vollständig in einem homozygoten Zustand deletiert ist, ist.

35. Transgenes Tier nach Anspruch 33, welches ein mutiertes Polynukleotid *KCNQ4* nach einem der Ansprüche 9, 11 oder 12 umfasst.

36. Transgenes Tier nach Anspruch 35, welches das mutierte Polynukleotid *KCNQ4* nach Anspruch 9 umfasst.

37. Transgenes Tier nach einem der Ansprüche 33-36, welches ein transgener Hamster, ein Meerschweinchen, ein Kaninchen oder eine Ratte ist.

38. Verwendung des transgenen Tiers nach einem der Ansprüche 33-37 zum Überprüfen von therapeutischen Verbindungen *in vivo.*

39. Verwendung nach Anspruch 38 zum Überprüfen von Arzneimitteln, die Krankheiten oder Zustände, die mit Gehörverlust oder Tinnitus verbunden sind, beeinflussen.

## Revendications

1. Polynucléotide isolé ayant une séquence d'acides nucléiques qui est capable de s'hybrider dans des conditions de haute stringence avec la séquence polynucléotidique présentée en tant que SEQ ID NO:1, ou son brin complémentaire, dans lequel ladite hybridation se produit dans une solution composée de SSC 5 x, solution de Denhardt 5X, SDS à 0,5% et 100 µg/ml d'ADN de sperme de saumon soniqué dénaturé pendant 12 heures à environ 45°C, suivie de deux lavages pendant 30 minutes dans du SSC 2X, SDS à 0,5% à une température d'au moins 65°C, codant pour une sous-unité KCNQ4 de canal potassique.

2. Polynucléotide isolé selon la revendication 1, dans lequel ladite hybridation est suivie de deux lavages pendant 30 minutes dans du SSC 2X, SDS à 0,5% à une température d'au moins 70°C.

3. Polynucléotide isolé selon la revendication 1, dans lequel ladite hybridation est suivie de deux lavages pendant 30 minutes dans du SSC 2X, SDS à 0,5% à une température d'au moins 75°C.

4. Polynucléotide isolé selon l'une quelconque des revendications 1 à 3, lequel est homologue au moins à 90% à la séquence polynucléotidique présentée en tant que SEQ ID NO:1.

5. Polynucléotide isolé selon la revendication 4, homologue au moins à 95% à la séquence polynucléotidique présentée en tant que SEQ ID NO:1.

6. Polynucléotide isolé selon l'une quelconque des revendications 1 à 5, lequel est un polynucléotide cloné.

7. Polynucléotide isolé selon la revendication 6, dans lequel le polynucléotide est cloné, ou produit à partir d'une banque d'ADNc.

8. Polynucléotide isolé selon l'une quelconque des revendications 1 à 7, comprenant la séquence polynucléotidique présentée en tant que SEQ ID NO:1.

9. Polynucléotide isolé selon l'une quelconque des revendications 1 à 7, comprenant la séquence polynucléotidique présentée en tant que SEQ ID NO:1, incluant la mutation G935A.

10. Polynucléotide isolé selon la revendication 1, codant pour la sous-unité du canal potassique KCNQ4 comprenant la séquence d'acides aminés représentée par SEQ ID NO:2.

11. Polynucléotide isolé selon la revendication 1, codant pour un variant de KCNQ4, lequel variant a une séquence d'acides aminés ayant été modifiée par délétion d'un résidu d'acide aminé, par insertion d'un résidu d'acide aminé supplémentaire, ou par substitution d'un résidu d'acide aminé au niveau d'une ou plusieurs positions situées dans les régions conservées, telles que définies par le Tableau 1.

12. Polynucléotide isolé selon la revendication 11, codant pour le variant G285S de SEQ ID NO:2.

13. Polypeptide produit par recombinaison codé par le polynucléotide selon l'une quelconque des revendications 1 à 12.

14. Polypeptide selon la revendication 13, lequel est une sous-unité de canal potassique KCNQ4 comprenant la séquence d'acides aminés présentée en tant que SEQ ID NO:2.

15. Polypeptide selon la revendication 13, lequel est un variant de KCNQ4, lequel variant a une séquence d'acides aminés ayant été modifiée par délétion d'un résidu d'acide aminé, par insertion d'un résidu d'acide aminé supplémentaire, ou par substitution d'un résidu d'acide aminé au niveau d'une ou plusieurs positions situées dans les régions conservées, telles que définies par le Tableau 1.

16. Polypeptide selon la revendication 15, lequel est le variant G285S de SEQ ID NO:2.

17. Cellule manipulée génétiquement par l'incorporation d'un polynucléotide hétérologue selon l'une quelconque des revendications 1 à 12.

18. Cellule selon la revendication 17, manipulée génétiquement par l'incorporation d'une sous-unité du canal KCNQ4 comprenant la séquence d'acides aminés présentée en tant que SEQ ID NO:2.

19. Cellule selon la revendication 18, manipulée génétiquement par l'incorporation d'un variant de KCNQ4, lequel variant a une séquence d'acides aminés ayant été modifiée par délétion d'un résidu d'acide aminé, par insertion d'un résidu d'acide aminé supplémentaire, ou par substitution d'un résidu d'acide aminé au niveau d'une ou plusieurs positions situées dans les régions conservées, telles que définies par le Tableau 1.

20. Cellule selon la revendication 19, manipulée génétiquement par l'incorporation du variant G285S de SEQ ID NO:2.

21. Cellule selon l'une quelconque des revendications 17 à 20, manipulée génétiquement pour co-exprimer une ou plusieurs sous-unités de canaux KCNQ.

22. Cellule selon la revendication 21, manipulée génétiquement pour co-exprimer le polypeptide KCNQ4 selon l'une quelconque des revendications 13 à 16 et les sous-unités du canal KCNQ1 ; le polypeptide KCNQ4 selon l'une quelconque des revendications 13 à 16 et les sous-unités du canal KCNQ2 ; le polypeptide KCNQ4 selon l'une quelconque des revendications 13 à 16 et les sous-unités du canal KCNQ3 ; le polypeptide KCNQ4 selon l'une quelconque des revendications 13 à 16 et les sous-unités des canaux KCNQ1 et KCNQ2 ; le polypeptide KCNQ4 selon l'une quelconque des revendications 13 à 16 et les sous-unités des canaux KCNQ1 et KCNQ3 ; le polypeptide KCNQ4 selon l'une quelconque des revendications 13 à 16 et les sous-unités des canaux KCNQ2 et KCNQ3 ; ou le polypeptide KCNQ4 selon l'une quelconque des revendications 13 à 16 et les sous-unités des canaux KCNQ1 et KCNQ2 et KCNQ3.

23. Cellule selon la revendication 22, manipulée génétiquement pour co-exprimer KCNQ3 et les sous-unités du canal KCNQ4 selon l'une quelconque des revendications 13 à 16.

24. Cellule selon l'une quelconque des revendications 17 à 23, laquelle est une cellule eucaryote, en particulier une cellule de mammifère, un ovocyte, ou une cellule de levure.

25. Cellule selon la revendication 24, laquelle est une cellule rénale embryonnaire humaine (HEK), une cellule HEK 293, une cellule BHK21, une cellule d'ovaire de hamster chinois (CHO), une cellule d'ovocyte de *Xenopus laevis* (XLO), ou toute autre lignée cellulaire capable d'exprimer les canaux potassiques KCNQ.

26. Procédé de criblage d'un composé chimique pour déterminer sa capacité à se lier à un canal potassique comprenant au moins une sous-unité du canal KCNQ4, lequel procédé comprend les étapes consistant à
(i) soumettre la cellule contenant la sous-unité du canal KCNQ4 selon l'une quelconque des revendications 17 à 25 à l'action d'un agent capable de se lier au polypeptide KCNQ4, pour ainsi former un complexe avec la cellule contenant la sous-unité du canal KCNQ4, dans lequel ledit agent capable de se lier au polypeptide KCNQ4 est
la 1,3-dihydro-1-phényl-3,3-bis(4-pyridylméthyl)-2H-indol-2-one (Linopirdine) marquée radioactivement ; ou
la 10,10-bis(4-pyridinyl-méthyl)-9(10H)-anthracénone marquée radioactivement.
(ii) soumettre le complexe de l'étape (i) à l'action du composé chimique à tester ; et
(iii) détecter le déplacement dudit agent capable de se lier au polypeptide KCNQ4 à partir du complexe avec la cellule contenant la sous-unité du canal KCNQ4.

27. Procédé selon la revendication 26, dans lequel ledit agent a été marqué radioactivement avec du ³H.

28. Procédé selon l'une ou l'autre des revendications 26 et 27, dans lequel le déplacement dudit agent capable de se lier au polypeptide KCNQ4 à partir du complexe avec la cellule contenant la sous-unité du canal KCNQ4 est détecté en mesurant la quantité de radioactivité par comptage par scintillation liquide classique.

29. Procédé de criblage d'un composé chimique pour déterminer l'activité sur un canal potassique comprenant au moins une sous-unité du canal KCNQ4, lequel procédé comprend les étapes consistant à
(i) soumettre une cellule contenant la sous-unité du canal KCNQ4 selon l'une quelconque des revendications 17 à 25 à l'action du composé chimique ; et
(iii) surveiller le potentiel de membrane, le courant, le flux potassique, ou l'influx calcique secondaire de la cellule contenant la sous-unité du canal KCNQ4.

30. Procédé selon la revendication 29, dans lequel la surveillance du potentiel de membrane de la cellule contenant la sous-unité du canal KCNQ4 est réalisée par des techniques de patch-clamp.

31. Procédé selon la revendication 30, dans lequel la surveillance du potentiel de membrane de la cellule contenant la sous-unité du canal KCNQ4 est réalisée au moyen de méthodes de fluorescence.

32. Utilisation d'une séquence polynucléotidique selon la revendication 9 pour le criblage de matériaux génétiques afin de dépister les individus ayant cette mutation.

33. Rongeur transgénique, porc transgénique, bovin transgénique, mouton transgénique ou chèvre transgénique comprenant une mutation knockout du polynucléotide *KCNQ4* endogène selon l'une quelconque des revendications 1 à 12, un polynucléotide *KCNQ4* muté selon l'une quelconque des revendications 9, 11 ou 12, ou manipulé génétiquement afin de surexprimer le polynucléotide *KCNQ4* selon l'une quelconque des revendications 1 à 12, ou afin de surexprimer le polynucléotide *KCNQ4* muté selon l'une quelconque des revendications 9, 11 ou 12.

34. Animal transgénique selon la revendication 33, lequel est un animal knockout chez qui le polynucléotide *KCNQ4* est totalement délété à l'état homozygote.

35. Animal transgénique selon la revendication 33, comprenant un polynucléotide *KCNQ4* muté selon l'une quelconque des revendications 9, 11 ou 12.

36. Animal transgénique selon la revendication 35, comprenant le polynucléotide *KCNQ4* muté selon la revendication 9.

37. Animal transgénique selon l'une quelconque des revendications 33 à 36, lequel est un hamster transgénique, un cobaye, un lapin ou un rat.

38. Utilisation de l'animal transgénique selon l'une quelconque des revendications 33 à 37 pour le criblage *in vivo* de composés thérapeutiques.

39. Utilisation selon la revendication 38, pour le criblage de médicaments agissant sur des maladies ou des affections associées à la perte d'audition ou aux acouphènes.
